(19) Europäisches Patentamt
European Patent Office
Office européen des brevets

(11) EP 0 640 616 B1

(12) **EUROPÄISCHE PATENTSCHRIFT**

(45) Veröffentlichungstag und Bekanntmachung des Hinweises auf die Patenterteilung:
**27.11.2002 Patentblatt 2002/48**

(51) Int Cl.7: **C07J 5/00**, A61K 31/57, C07J 17/00, C07J 33/00, C07J 43/00, C07J 41/00, C07J 31/00

(21) Anmeldenummer: **94113048.6**

(22) Anmeldetag: **22.08.1994**

(54) **Corticosteroid-17-alkylcarbonat-21-(O)-Carbonsäure- und Kohlensäureester, Verfahren zu deren Herstellung und diese enthaltende Arzneimittel**

17-Alkylcarbonate, 21-0-carbonic/carboxylic acid esters of corticosteroids, a method for their production and pharmaceutical compositions containing them

Dérivés 17-alkylecarbonate, 21-0-esters des corticostéroides, un procédé de leur préparation et les compositions pharmaceutiques les contenant

(84) Benannte Vertragsstaaten:
**AT BE CH DE DK ES FR GB GR IE IT LI LU NL PT SE**

(30) Priorität: **27.08.1993 DE 4328819**

(43) Veröffentlichungstag der Anmeldung:
**01.03.1995 Patentblatt 1995/09**

(73) Patentinhaber: **HOECHST AKTIENGESELLSCHAFT**
**65929 Frankfurt am Main (DE)**

(72) Erfinder:
- **Stache, Ulrich, Dr.**
  **D-65719 Hofheim (DE)**
- **Alpermann, Hans-Georg, Dr.**
  **D-61462 Königstein (DE)**
- **Dürckheimer, Walter, Dr.**
  **D-65795 Hattersheim (DE)**
- **Bohn, Manfred, Dr.**
  **D-65719 Hofheim (DE)**

(56) Entgegenhaltungen:
**EP-A- 0 000 742** **EP-A- 0 072 200**
**EP-A- 0 156 643**

- **THE JOURNAL OF STEROID BIOCHEMISTRY AND MOLECULAR BIOLOGY, Bd.44, Nr.2, Februar 1993, OXFORD, GB Seiten 141 - 145 M. ISOGAI ET AL 'Binding affinities of mometasone furoate and related compounds including its metabolites for the glucocorticoid receptor of rat skin tissue.'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Corticoid-17-alkylcarbonat-21-Carbonsäure- und Kohlensäureester der Formel I

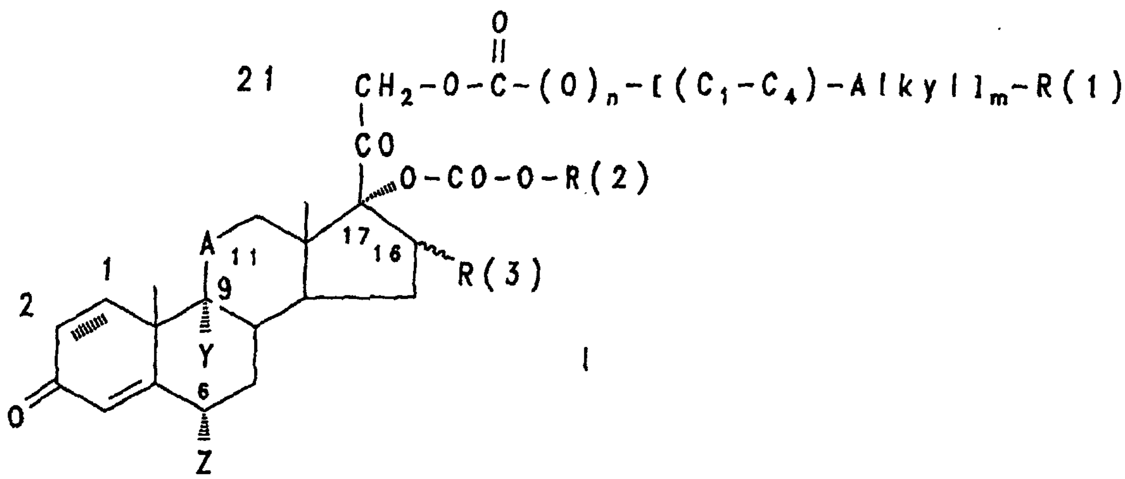

I

in welcher bedeuten:

A CHOH und CHCl in beliebiger sterischer Anordnung, $CH_2$, C = O, 9(11)-Doppelbindung;
Y Wasserstoff, Fluor oder Chlor;
Z Wasserstoff, Fluor oder Methyl;
R(1) Phenyl, Pyridyl, Thienyl, Pyrrolyl, Furyl, Indolyl oder Fluorenyl, ($C_1$-$C_4$)-Alkyl gesättigt, einfach oder mehrfach ungesättigt;
n Null oder 1,
m Null oder 1,
R(2) lineares oder verzweigtes ($C_1$-$C_8$)-Alkyl, $-(CH_2)_2-OCH_3$
R(3) Wasserstoff, α- oder β-Methyl.

Bevorzugt sind Verbindung der Formel I, in welchen bedeuten:

R(1), A, Y, Z und R(3) wie in Anspruch 1 definiert; und
R(2) lineares oder verzweigtes ($C_1$-$C_5$)-Alkyl, $-(CH_2)_2-OCH_3$.

Besonders bevorzugt sind Verbindungen der Formel I, in welchen R(1) gleich Phenyl ist.
Ganz besonders bevorzugt sind Verbindungen der Formel I, die ausgewählt sind aus der Gruppe bestehend aus

Prednisolon-17-ethylcarbonat-21-benzoat,
Prednisolon-17-ethylcarbonat-21-phenylacetat,
Prednisolon-17-ethylcarbonat-21-(3)-phenylpropionat,
Prednisolon-17-ethylcarbonat-21-(thienyl-2-essigsäure)ester,
Prednisolon-17-ethylcarbonat-21-(thiophen-2-carbonsäure)ester,
Prednisolon-17-ethylcarbonat-21-(furan-2-carbonsäureester),
Prednisolon-17-n-propylcarbonat-21-(3)-phenylpropionat,
Prednisolon-17-n-propylcarbonat-21-phenylacetat,
Prednisolon-17-iso-propylcarbonat-21-phenylacetat,
Prednisolon-17-n-butylcarbonat-21-phenylacetat,
Betamethason-17-ethylcarbonat-21-(3)-phenyl-propionat,
Betamethason-17-ethylcarbonat-21-phenylacetat,
Betamethason-17-ethylcarbonat-21-(thienyl-2-essigsäure)ester,
Betamethason-17-ethylcarbonat-21-(furan-2-carbonsäureester),
Betamethason-17-ethylcarbonat-21-(indol-3-essigsäure)ester,

Prednisolon-17-ethylcarbonat-21-(indol-3-essigsäure)ester,
Dexamethason-17-ethylcarbonat-21-phenylacetat,
Hydrocortison-17-ethylcarbonat-21-phenylacetat,
Cortison-17-ethylcarbonat-21 -phenylacetat,
6α-Methylprednisolon-17-ethylcarbonat-21-phenylacetat,
Prednison-17-ethylcarbonat-21-phenylacetat,
6α-Fluor-prednisolon-17-ethylcarbonat-21-phenylacetat,
6α-Fluor-dexamethason-17-ethylcarbonat-21-phenylacetat,
6α-Fluor-betamethason-17-ethylcarbonat-21-phenylacetat,
6α,16α-Dimethyl-prednisolon-17-ethylcarbonat-21-phenylacetat,
17α-Ethylcarbonat, 21-phenylacetat von Reichsteins Substanz S,
Beclomethason-17α-ethylcarbonat-21-phenylacetat,
6α-Methyl-9α-fluor-prednisolon-17-ethylcarbonat-21-phenylacetat,
Betamethason-17-n-propylcarbonat-21-phenylacetat,
Dexamethason-17-iso-propylcarbonat-21-phenylacetat,
Prednisolon-17-n-propylcarbonat-21-phenylacetat,
Prednisolon-17-iso-propylcarbonat-21-phenylacetat,
Prednisolon-17-n-butylcarbonat-21-phenylacetat,
Prednisolon-17-iso-butylcarbonat-21-phenylacetat,
Prednisolon-17-methoxyethylcarbonat-21-phenylacetat, und
Prednisolon-17-ethylcarbonat-21-phenylcarbonat,

Ganz besonders speziell bevorzugt sind die Verbindungen Prednisolon-17-ethylcarbonat-21-phenylacetat oder
Betamethason-17-ethylcarbonat-21-phenylacetat.

Die Erfindung betrifft auch ein Verfahren zum Herstellen einer Verbindung I, bei welchem man

a) eine Verbindung der Formel II,

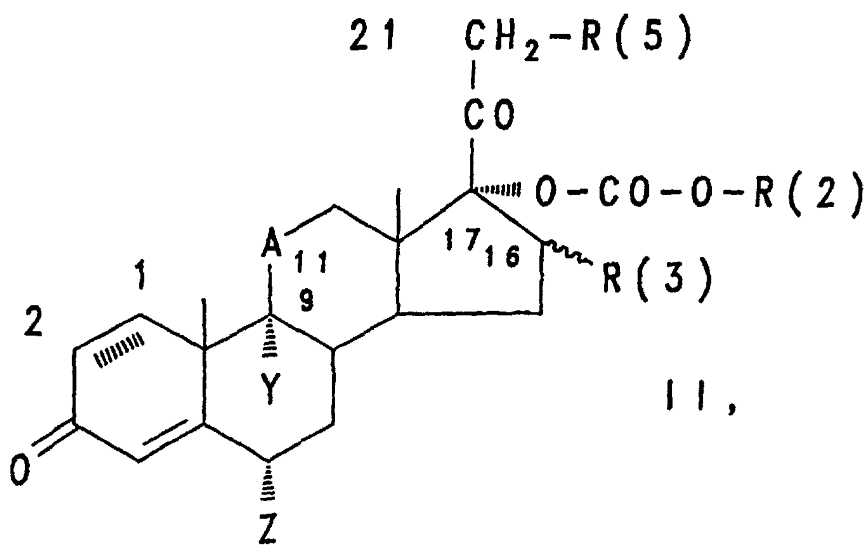

II,

in der R(5) gleich OH ist und die übrigen Substituenten die oben angegebenen Bedeutungen haben,

a 1) mit einer aktivierten Carbonsäure der Formel III, vorzugsweise einem Halogenid oder Anhydrid oder Azolid,

$$R(6)\text{-}CO\text{-}(O)_n\text{-}[(C_1\text{-}C_4)\text{-}Alkyl]_m\text{-}R(1) \qquad III$$

umsetzt, wobei bedeuten:

n Null,
m Null oder 1, und

$[(C_1-C_4)$-Alkyl] und R(1) die oben angegebenen Bedeutungen haben und

R(6) Cl, Br, O[-CO-$(O)_n$-$[(C_1-C_4)$-$Alkyl]_m$-$R(1)]_1$-, -OC(O)$CF_3$ oder ein anderes aktiviertes Säureradikal, oder

a 2) mit einem Halogenformat der Formel III,
in der

n 1,
m Null oder 1,

$[(C_1-C_4)$-Alkyl] und R(1) die oben angegebenen Bedeutungen haben und R(6) Cl, Br, J bedeuten, oder

a 3) mit einer Carbonsäure der Formel III selbst, in der

R(6) OH und
n Null sind,

und die weiteren Substituenten bei Formel III angegeben sind,

in Gegenwart Wasser abspaltender Reagentien (DCC etc.) umsetzt
oder daß man
b) Verbindungen der Formel II,

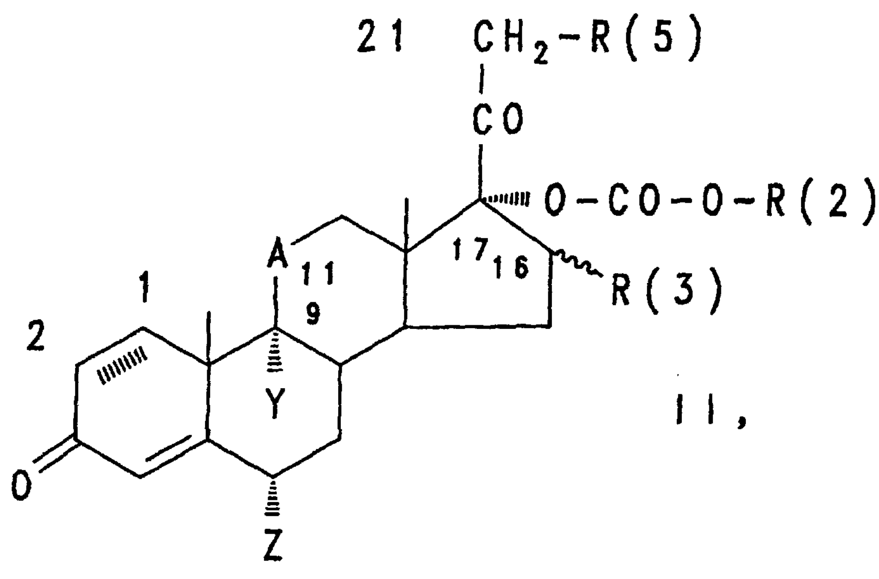

II,

in der R(5) = Br, J, eine Sulfonsärearyl- oder -alkylestergruppierung ist und die weiteren Substituenten die bei Formel I angegebenen Bedeutung haben, mit einem Salz, vorzugsweise K- oder Na-Salz oder einem Trialkylammoniumsalz, einer Carbonsäure der Formel III,

$$R(6)\text{-}CO\text{-}(O)_n\text{-}[(C_1\text{-}C_4)\text{-}Alkyl]_m\text{-}R(1) \qquad III$$

in der

R(6) -[$O^-Me^+$] und
n Null bedeuten,

und die weiteren Substituenten die bei Formel III angegebenen Bedeutungen haben,
umsetzt,
wobei Me vorzugsweise das Kation eines Alkalisalzes oder eines Trialkylammoniumsalzes ist.

Aus der EP 742 sind Bis-alkyl-Derivate von Steroiden bekannt; ein Hinweis auf 17,21-bis-Arylderivate findet sich dort nicht. J. steroid Biochem. Molec. Biol. 44/2, 141 (1993) beschreibt nur Steroide, die weder in 17-stellung ein Carbonat, noch in 21-Stellung einen Aromaten tragen. Beide stellen nehmen die Erfindung weder vorweg, noch legen sie sie nahe.

Die als Ausgangssubstanzen benötigten Steroid-17-alkylcarbonate mit freier 21-Hydroxylgruppe der Formel II [R(5) = OH] werden nach den Verfahren der US-Patentschrift 4 242 334 (HOE 77/F 154) und der europäischen Offenlegungsschrift 470 617 (HOE 90/F 241) hergestellt.

Die Steroid-17-alkylcarbonate mit R(5) gleich Br, J, -$OSO_2$-Aryl, -$OSO_2$-Alkyl in Formel II werden nach der US-Patentschrift 4 377 575 (HOE 78/F 082) und der europäischen Offenlegungsschrift 470 617 (HOE 90/F 241) hergestellt. Hierbei kommen die 17-Alkylcarbonate folgender Costicosteroide in Frage:

Prednisolon, Prednison, 6α-Methyl-predniolon, 6α,16α-Dimethylprednisolon, 16α-Methyl-prednisolon, Hydrocortison (Cortisol), Cortison, 6α-Methyl-cortisol, Reichsteins Substanz S, 11-Desoxi-9(11)-dehydro-prednisolon, 6α-Fluorprednisolon, Dexamethason, 6α-Fluor-dexamethason, 9α-Fluorprednisolon,, 6α,9α-Difluor-prednisolon, 6α-Methyl,9αfluor-prednisolon, Betamethason, Clobetasol.

Die als Reaktionspartner zum Einsatz kommenden Carbonsäuren der Formel III [R(6) gleich OH und n gleich Null] bzw. deren aktivierte Derivate, wie die Halogenide [R(6) = Cl, Br, J oder deren Anhydride], oder deren Azolide [R (6) gleich Imidazolid, Triazolid] oder deren Salze [R(6) gleich (MeO)-, vorzugsweise (KO)-, (NaO)-] sind in der Regel bekannt und werden ggf. nach allgemeinen präparativen Methoden hergestellt. Beispiele der gemäß der Erfindung zum Einsatz gelangenden Carbonsäuren gemäß Formel III [R(6) gleich OH und n gleich Null] findet man in der Liste am Ende des Textes vor den Ansprüchen.

Alle hierunter fallenden Carbonsäuren tragen in ihrem Säurerest eine Aryl- oder Hetarylgruppe. Letztere sind essentieller Bestandteil der Erfindung.

Die punktierte Linie zwischen den C-Atomen 1 und 2 zeigt an, daß diese Bindung eine Einfachbindung oder eine ungesättigte Bindung sein kann.

Wie im pharmakologischen Teil gezeigt wird, zeigen insbesondere Corticoid-17-alkylcarbonat-21-carbonsäureester dieses Typs (= 21-Aryl- bzw. -Hetarylester-Typ) im Vergleich zu strukturverwandten Corticoid-17-alkylcarbonat-21-carbonsäureestern, die keine Aryl- bzw. Hetarylgruppe im 21-Säurerest tragen oft deutlich bessere Wirkqualitäten hinsichtlich des Verhältnisses lokale/systemische antiinflammatorische Wirkung.

Detaillierte Beschreibung der einzelnen Reaktionsführungen der Herstellungsverfahren für die erfindungsgemäßen Verfahrensprodukte gemäß Formel I:
zu Verfahrensvariante a:

Zur Herstellung von 21-Carbonsäureestern des o. a. Typs werden vorzugsweise entweder Carbonsäurehalogenide oder -azolide der Formel IV

$$R(6)\text{-OC-}[(C_1\text{-}C_4\text{-Alkyl}]_m\text{-}R(1) \qquad \text{IV},$$

in der bedeuten:

R(6) Cl, Br, J,

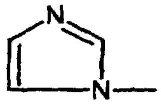

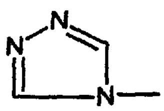

m Null oder 1 und

R(1) sowie ($C_1$-$C_4$)-Alkyl die zur Formel III angegebenen Bedeutungen haben

oder Carbonsäureanhydride der Formel V

$$O\{-OC-[(C_1-C_4)-Alkyl]_m-R(1)\}_2 \qquad V,$$

in der bedeuten:

m Null oder 1, und

R(1) sowie $(C_1-C_4)$-Alkyl die zur Formel III angegebenen Bedeutungen haben, verwendet. In beiden Fällen können die ihnen zugrundeliegenden in der Liste aufgeführten Carbonsäuren verwendet werden, vorzugsweise deren Carbonsäurechloride, -anhydride und -imidazolide bzw. -triazolide.

R(6) in Formel IV können auch andere die Carboxylgruppe in Carbonsäuren die Veresterung aktivierende Gruppen beinhalten, so beispielsweise $-O-CO-CF_3$ oder die aus Phosphon- oder Phosphinsäureanhydriden (z. B. Propanphosphonsäureanhydrid) oder Polyphosphorsäureanhydrid (PPA) herstellbaren aktivierten Carbonsäuren.

Weitere Phosphorreagentien, die eine schonende Veresterung von organischen Carbonsäuren mit der 21-Alkoholgruppe von Corticoid-17-alkylcarbonaten bewirken können sind in den Literaturstellen Synth. Commun. 13, 471ff (1983) und Synth. Commun. 14, 515ff (1984) angeführt bzw. beschrieben.

Zur Veresterung mit einem Carbonsäurehalogenid oder -anhydrid oder einem Halogenformat löst man die Steroidkomponente in einem inerten Lösungsmittel, beispielsweise in einem Ether, wie Dioxan, Tetrahydrofuran, Diglym, oder gegebenenfalls halogenierten Kohlenwasserstoffen, wie Benzol, Toluol, Cyclohexan, Methylenchlorid, Chloroform oder in Aceton oder in einem Gemisch dieser Lösungsmittel. Zur Entfernung der in der Reaktion entstehenden Halogenwasserstoffsäure setzt man 1 - 1000 Moläquivalente einer tertiären Base, wie Pyridin, Chinolin, Triethylamin, Dimethylanilin, Dimethylaminopyridin usw., zu. Man kann aber auch eine anorganische Base, wie Natriumhydrogencarbonat oder Calciumcarbonat, zur Entfernung der Säure benutzen. Anschließend tropft man 1 - 200 Moläquivalente, vorzugsweise 1 - 3 Moläquivalente eines der oben angeführten Acylierungsmittel, gegebenenfalls gelöst in einem der oben angeführten Lösungsmittel, bei einer Temperatur von -40°C bis zum Siedepunkt des verwendeten Lösungsmittels, vorzugsweise von 0°C bis 25°C, zu. Anschließend läßt man das Reaktionsgemisch eine bis 120 Stunden bei einer Temperatur von -40°C bis zum Siedepunkt des Lösungsmittels, vorzugsweise von 0°C bis 25°C stehen.

Bei Verwendung von Carbonsäureanhydriden als Acylierungsmittel ist es hin und wieder von Vorteil, ohne Zusatz von Lösungsmitteln zu arbeiten. Es reicht in der Regel aus, lediglich die organische Base, vorzugsweise Pyridin, dem gegebenenfalls im Überschuß angewandten Säureanhydrid zuzufügen.

Insbesondere bei empfindlichen (und zuweilen instabilen) Carbonsäurederivaten des o. a. Typs, insbesondere bei Verwendung von Phenylacetylchloriden, -anhydriden, Hetarylacetylchloriden und -anhydriden, ist es von großem präparativem und reaktionsselektivem Vorteil, die Corticoid-17-alkyl-carbonate mit freier 21-Hydroxylgruppe mit 1 - 4 Moläquivalenten des Chlorids bzw. Anhydrids bei -10° bis +6° (maximal 20°C) in chlorierten Kohlenwasserstoffen, wie vorzugsweise Dichlormethan, sowie mit 1 - 4 Moläquivalenten einer Pyridinbase, vorzugsweise Dimethylaminopyridin, umzusetzen.

Hierbei werden die Reaktionsprodukte der Formel I in hoher Reinheit, ohne nennenswerte Beimengungen an Nebenprodukten, insbesondere 11-acylierten Produkten, erhalten (Verfolgung der Reaktionsführungen durch DC), das heißt die Reaktionsführungen sind hinsichtlich der Umsetzung der 21-Hydroxygruppe hoch regioselektiv.

Bei den Reaktionen mit Carbonsäurechloriden wird in vorteilhafter Weise oft absolutes Dioxan oder Tetrahydrofuran zum Reaktionsgemisch gegeben, z. B. bei Benzoylchlorid, wobei das Verhältnis Dioxan/Pyridin etwa 1 : 1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).

Die Charakterisierung der Reaktionsprodukte kann durch Dünnschicht-Chromatographie (DC) erfolgen; hierbei haben die Reaktionsprodukte $R_F$-Werte von etwa 0.65 - 0.8. In der Regel werden die Reaktionsprodukte durch Massenspektren mit MS = m/z = .... $(M + H^+)$ charakterisiert (in der Regel FAB-Spektren); es werden jeweils die monoisotopischen Molmassen erfaßt. Die $M + H^+$-Werte wurden jeweils aufgerundet. Auch IR-, $^1H$-NMR- und UV-Spektren können zur Charakterisierung herangezogen werden.

Zur Aufarbeitung gießt man das Reaktionsgemisch in Wasser, das gegebenenfalls mit Natriumchlorid und Natriumbicarbonat versetzt wurde, wobei die Reaktionsprodukte, oft erst nach längerem Stehen, im allgemeinen kristallin ausfallen. Ölig oder wachsartig gebliebene Reaktionsprodukte werden durch Ausschütteln mit einem geeigneten Extraktionsmittel und Eindampfen angereichert. Die Reaktionsprodukte können, falls erforderlich, durch Umkristallisieren oder durch Chromatographie aufgetrennt oder gereinigt werden. Oft genügt auch intensives Digerieren in einem das Reaktionsprodukt möglichst wenig oder nicht lösenden organischen Lösungsmittel, wie Diethylether oder Cyclohexan, oder einem Gemisch aus diesen Komponenten zur weiteren Reinigung der Reaktionsprodukte.

Bei Verwendung von Carbonsäureazoliden führt man die Veresterung zweckmäßig als Eintopfreaktion durch. Hierbei löst man beispielsweise Aryloder Hetarylessigsäure oder eine andere Carbonsäure der Formel III [R(6) gleich OH), n gleich Null], in absolutem Pyridin und gibt eine vorzugsweise äquimolare Menge N,N-Carbonyl-diimidazol oder -[1H-1,2,4-triazol] hinzu, wobei sich bei 0°-20° die entsprechenden Säureazolide bilden. Nach Zugabe einer etwa äquimolaren Menge Corticoid-17-alkylcarbonat der Formel II [R(5) = OH] und katalytischer Menge einer Base, vorzugsweise Natriumhydrid oder -imidazolid rührt man in Pyridin zwischen 0° bis 40°C; vorzugsweise 20° und arbeitet wie üblich auf.

Man kann aber auch das vorher durch äquimolare Mengen N,N'-Carbonylazolid und Carbonsäure in absolutem Tetrahydrofuran hergestellte und isolierte Carbonsäureazolid in Lösungsmitteln wie Pyridin, Dimethylformamid, Tetrahydrofuran im gelösten Steroid zugeben und weiter wie oben geschildert verfahren [s. auch Chem. Ber. 95, S. 1284 ff. (1962)].

Bei der Veresterung mit Hilfe von Phosphon- bzw. Phosphinsäureanhydriden setzt man vorzugsweise äquimolare Mengen Carbonsäure und Corticoid-21-alkohol in absolutem Pyridin mit 50 %igen Propanphosphorsäureanhydrid in Methylenchlorid bei 20° bis 60° unter Zugabe von 4-Dimethylaminopyridin als Säurefänger hinzu und arbeitet wie üblich auf (in Eiswasser eingießen, mit Essigester extrahieren, mit 5 % $KHSO_4$ waschen, abdestillieren, kristallisieren). Anstelle von Phosphonsäureanhydriden kann man auch Polyphosphorsäureanhydrid (PPA) einsetzen.

Ein weiteres vorteilhaftes Veresterungsverfahren, das auf die gemäß Formel III [R(6) gleich OH und n gleich Null] oder in der Liste aufgeführten Carbonsäuren anwendbar ist, ist die direkte Umsetzung von Corticoid-17-Alkylcarbonaten der Formel II [R(5) gleich OH] mit Hilfe von wasserentziehenden Mitteln, wie Carbodiimiden, vorzugsweise N,N'-Dicyclohexylcarbodiimid (DCC). Anstelle von DCC kann man in einigen Fällen auch mit "Molekularsieben" als wasserentziehenden Mitteln arbeiten.

Durch Zusatz einer Säure, z. B. Schwefelsäure, Phosphorsäure, Chlorwasserstoffsäure, Diphenylphosphorsäure, p-Toluolsulfonsäure bzw. von deren Pyridiniumsalzen oder einer organischen Base, wie z. B. Dimethylaminopyridin (= besonders vorteilhaft in halogenierten Lösungsmitteln, wie z. B. Methylenchlorid, oder in Dimethyl-formamid) kann die Veresterung katalytisch beschleunigt bzw. optimiert werden, was insbesondere bei sonst schwer reagierenden bzw. empfindlichen Carbonsäuren, z. B. vom Indolylessigsäure-, Pyrrolcarbonsäure-, Aryl- und Hetarylessigsäuretyp usw. sehr vorteilhaft ist. Hierbei ist es überraschend, daß die sekundäre 11-Hydroxygruppe in den eingesetzten Corticoid-17-alkylcarbonaten praktisch in der Regel nicht gleichzeitig mit verestert wird, wie man es öfters bei der Veresterung mit den entsprechenden Säurehalogeniden beobachtet.

In einer besonderen Verfahrensvariante gibt man zu einer Lösung von 1 Moläqu. Corticoid-17-alkylcarbonat-21-alkohol [Formel II, R(5) gleich OH] und 1 - 4 Moläqu. Carbonsäure der Formel III [R(6) gleich OH, n gleich Null] vorzugsweise 2 Äquivalente, in absolutem Pyridin eine katalytische Menge Schwefelsäurepyridiniumsalz, sowie nach circa 20 Min. 1 - 4 Moläqu. Dicyclohexylcarbodiimid, vorzugsweise 1 - 2 Moläqu. zu. Man rührt hiernach bei 0° - 50°C, vorzugsweise 20°C, bis eine DC-Probe keine Ausgangscarbonsäure, sondern nur gewünschte Carbonsäure-21-corticoidester der Formel I anzeigt. Man filtriert vom entstandenen Dicyclohexylharnstoff ab, gießt das Filtrat zweckmäßig in Wasser ein, filtriert (bei Kristallbildung) oder dekantiert (bei öligen bzw. wachsartigen Umfällungen) ab, wäscht mit Wasser nach (ggf. extrahiert man auch mit Extraktionsmittel, insbesondere Dichlormethan) trocknet, kristallisiert wie üblich um oder stellt, falls erforderlich, die Reaktionsprodukte durch übliche Chromatographie, vorzugsweise an Kieselgel, rein dar.

Anstelle von Pyridin können in einigen Fällen auch anderen inerte Lösungsmittel, wie z. B. Tetrahydrofuran, Dioxan, Methylenchlorid, Dimethylformamid zweckmäßig unter Hinzugabe von tertiären Basen, beispielsweise Pyridin, 4-Dimethylaminopyridin verwendet werden. Bei Verwendung von Molekularsieben als wasserentziehenden Mittel sind letztere Lösungsmittel vorzuziehen.

Für die Veresterung mit den empfindlichen Aryl- und Hetarylessigsäuren hat sich weiterhin folgende Variante bewährt: 1 Äqu. Carbonsäure wird bei 0° C in absolutem Dichlormethan gelöst, und nacheinander wird mit 1 Äqu. DCC, 0.2 Äqu. 4-N,N'-Dimethylaminopyridin und einer Lösung von 1 Äqu. Corticosteroid-17-alkylcarbonat-21-alkohol in absolutem Dichlormethan versetzt und 18 bis 48 Std. bei 20°C gerührt. Nach üblicher Aufarbeitung kann der gewünschte Ester der Formel I rein dargestellt werden. Anstatt DCC kann auch Molekularsieb verwendet werden.

In einer weiteren Veresterungsmethode wird Corticoid-17-alkylcarbonat-21-[tert.-Butyldimethylsilyl-(O)-ether] in absolutem Tetrahydrofuran mit 1 Moläqu. Carbonsäure und Trifluoressigsäureanhydrid versetzt, und nach etwa 1 - 6 Std. Rühren wird bei 20° C wie üblich aufgearbeitet.

Man kann aber auch direkt die Carbonsäure sowie den Corticoid-17-alkylcarbonat-21-alkohol (freie Form) mit Trifluoressigsäureanhydrid zum gewünschten 21-Carbonsäureester umsetzen (= Bildung des gemischten Anhydrids aus Carbonsäure und Trifluoressigsäure, das dann mit dem 21-Alkohol zum 21-Ester reagiert).

zu Verfahrensvariante b:

Eine weitere vorteilhafte Verfahrensvariante, die zu den erfindungsgemäßen Corticoiden führt, besteht darin, daß man ein(en) Corticoid-17-alkylcarbonat-21-halogenid, vorzugsweise 21-Iodid oder 21-Bromid oder 21-Sulfonat, vorzugsweise 21-p-Chlorbenzolsulfonsäureester oder 21-methansulfonsäureester mit den Metallsalzen, vorzugsweise

Alkalisalzen oder Trialkylammoniumsalzen, der in Liste 2 aufgeführten Carbonsäuren in inerten organischen Lösungsmitteln, vorzugsweise Dimethylsulfoxyd, Dimethylformamid, Butanon-(2), Aceton, Acetonitril, 1 - 16 Std., vorzugsweise 1 - 10 Std. bei 20°C bis zu den Siedepunkten der verwendeten Lösungsmittel, vorzugsweise ca. 50°C, erhitzt und nach üblicher Aufarbeitung, vorzugsweise Eingießen von Wasser, Abfiltrieren oder Abdekantieren des Niederschlags und üblicher Reindarstellung isoliert.

Bei dieser nucleophilen Austauschreaktion einer 21-Halogenid- bzw. 21-Sulfonsäureestergruppe gegen eine Carbonsäureestergruppe ist es überraschend, daß unter den vorzugsweise alkalischen Reaktionsbedingungen die für das Wirkungsprofil mitverantwortliche 17-Alkylcarbonatgruppe in den Verfahrensprodukten nicht gleichzeitig verseift wird.

Für die gemäß Verfahrensweisen a) und b) hergestellten Verbindungen I gilt, daß eine Hydroxygruppe in 11-Stellung gegebenenfalls nach üblichen Methoden zur Ketogruppe oxydiert werden kann. Vorzugsweise wird diese Oxydation mit Chromtrioxid in saurem Medium und in einem inerten organischen Lösungsmittel durchgeführt. Eine im Corticoidteil vorhandene 9(11)-Doppelbindung kann gegebenenfalls durch Addition von Halogenwasserstoffsäure oder durch Chlor nach üblichen bekannten Methoden in die entsprechenden erfindungsgemäßen Corticoid-17-alkylcarbonat-21-ester mit einer 11β-Hydroxyl-9α-Halogenidgruppe (9αF,Cl) oder 11β-9α-Dichlorgruppe überführt werden.

Die Verfahrensprodukte besitzen wertvolle pharmakologische Eigenschaften. Sie sind insbesondere lokal und topisch sehr stark antiphlogistisch wirksam und zeigen teilweise ein überraschend sehr gutes Verhältnis von lokaler zu systemischer antiinflammtorischer Wirkung, das gegenüber analogen Corticoid-17-alkylcarbonat-21-estern, die im 21-Esterrest keine Aryl- oder Hetarylgruppe tragen, so z. B. 21-Estergruppen mit einer 21-Alkylgruppe, oft deutlich überlegen ist, wie aus pharmakologischen Standardtests hergeleitet werden kann (siehe Pharmakolog. Test-Teil). Demgemäß ist Gegenstand der Erfindung auch ein Mittel zur Behandlung entzündlicher Dermatosen bestehend aus einer Verbindung der Formel I.

Die Verfahrensprodukte können in der Veterinär- und Humantherapie zur Behandlung von entzündlichen Dermatosen verschiedenster Genese in Form von Suspensionen, Salben, Cremes, Sprays usw. Verwendung finden. Dabei ist als besonders vorteilhaft für die lokale und topische Therapieform herauszuheben, daß die Verfahrensprodukte aufgrund ihres äußerst günstigen Verhältnisses von lokaler zu systemischer antiphlogistischer Wirkung auch bei hochdosierter und langanhaltender Therapie praktisch nur geringfügige systemische Nebenwirkungen hervorrufen können. Bei äußerlicher Behandlung werden Salben, Cremes, Suspensionen usw. mit einer Konzentration von 0.01 bis 2 Gew.-% verwendet. Insbesondere zeigen die Verfahrensprodukte in pharmakologischen Tests einen zum Teil wesentlich besseren Split (Verhältnis) von lokaler/systemischer antiinflammatorischer Wirkung als entsprechende Präparate mit einer 21-Estergruppe, die im Esterteil keine Aryl- bzw. Hetarylanteile, wie es bei den erfindungsgemäßen Verbindungen der Fall ist, aufweisen. Weiterhin zeigen die Verfahrensprodukte teilweise auch eine stärkere lokale antiphlogistische Wirksamkeit als die zuletzt genannten Analogpräparate. Darüber hinaus können die erfindungsgemäßen Corticoid-17-alkylcarbonat-21-ester gegenüber den analogen zuletzt genannten Corticoid-17-alkylcarbonat-derivaten oft eine noch geringere Haut- Atrophogenität aufweisen, was ein weiterer Vorteil für eine dermatotherapeutische Behandlung ist.

Darüber hinaus können die erfindungsgemäßen Verfahrensprodukte mit diversen gut hautverträglichen lokal wirksamen Antibiotika, z. B. vom Typ des Gentamycins, Neomycins, Erythromycins, Tetracyclins oder der Fusidinsäure und anderen, in galenischen Formulierungen kombiniert werden. Derartige Kombinationen aus den Verfahrensprodukten und den lokalen wirksamen Antibiotika können zur Behandlung von primären bakteriellen oder bakteriell superinfizierten entzündlichen Dermatosen verwendet werden.

Pharmakologischer Versuchsteil

So zeigten z. B. Prednisolon-17-ethylcarbonat-21-phenylessigsäure-ester (I) oder Betamethason-17-ethylcarbonat-21-phenylessigsäure-ester (II) eine starke lokale antiphlogistische Wirkung bei einem auffallend günstigen Split zur schwach systemischen Wirksamkeit, wie aus den unten angeführten pharmakologischen Testergebnissen [Vergleichspräparat Prednicarbat (= Prednisolon-17-ethylcarbonat-21-propionat (US-Patentschrift 4 242 334) und (Merck Index 11, 7717))] hervorgeht:

1. Lokale antiphlogistische Wirkung im Crotonöl-Ohrödem an Ratten nach epikutaner Application

Wir verwendeten die Rattenohr-Methode von Tonelli et al.: Männliche Wistar-Ratten aus eigener Zucht im Gewicht um 50 g wurden am rechten Ohr mit dem Irritans bzw. Testsubstanz enthaltenden Irritans epikutan behandelt. Das linke Ohr blieb unbehandelt. Zur Entzündungsauslösung diente TPA (12-o-Tetradecanoylphorbol-13-acetat, SIGMA P 8139) in Aceton gelöst, 0,4 mg/ml (davon je 20 µl innen bzw. außen). Die zu prüfenden Kortikoide wurden hierin in den angegebenen Endkonzentrationen gelöst. Kontrollen erhielten nur das TPA-Lösungsmittelgemisch. 4 h nach epikutaner Behandlung wurden die Tiere mit $CO_2$ getötet. Aus dem rechten (behandelten) und dem linke (unbehandelten) Ohr wurden 8 mm Durchmesser messende Scheiben ausgetanzt und sofort gewogen. Diese Differenz als Parameter für den Grad der Entzündung bei Kontrollen (mg, x ± s) wurde gleich 100 gesetzt. Die antiphlogistische Wirkung wird durch Angabe der ca. 50 %igen Hemmdosis in mg/ml charakterisiert:

| Behandlung | mg/ml | x ± s (mg) | Hemmung in % |
|---|---|---|---|
| Kontrolle | | 15 ± 5.2 | - |
| Verb. I | 0.03 mg/ml | 10 ± 3.0 | 33 |
| Verb. I | 0.1 mg/ml | 5 ± 1.1 | 67 |
| Verb. I | 0.3 mg/ml | 3 ± 2.3 | 80 |
| Verb. II | 0.1 mg/ml | 5.1 ± 3.3 | 66 |
| Verb. II | 0.3 mg/ml | 4.5 ± 3.1 | 70 |
| Prednicarbat | 1 mg/ml | 3 ± 1.7 | 80 |
| Ergebnis:<br>Verb. I ist ca. dreimal stärker als Prednicarbat,<br>Verb. II entspricht der Wirkung von Verb. I | | | |

2 a) Prüfung auf systemische antiphlogistische Wirkung im Test "Antiphlogistische Wirkung nach subcutaner Gabe: Carrageenan-pfotenödem an Ratten".

Als Test für die akute systemische antiphlogistische Wirkung wurde das Carrageenan-Pfotenödem an Ratten nach der von Winter et al. Proc. Soc. Exp. Biol. (New York) Band 111, S. 544 (1962) beschriebenen Methode gewählt. Männliche Sprague-Dawley-Ratten im Gewicht um 120 g erhielten die zu prüfenden Substanzen s.c. (0.2 ml/100 g) in Sesamöl gelöst. 30 min später wurde in die linke Hinterpfote 0.1 ml einer 0.5 % Carrageenan-Lösung injiziert. 6 h später wurde die Schwellungszunahme volumetrisch gemessen. Kontrollen erhielten nur Sesamöl.

Die Pfotenvolumina sind in ml, x ± s, angegeben. Die antiphlogistische Wirkung wird auch hier durch Angabe der ca. 50 %igen Hemmdosis in mg/kg charakterisiert.

| Dosis in mg/kg Ausgangswert Volumenzunahme | | | |
|---|---|---|---|
| | s.c. | (ml) | (ml) |
| | | x ± s | (x ± s) |
| Kontrolle | Sesamöl | 1.44 ± 0.09 | 0.57 ± 0.07 |
| Verb. I | 0.3 | 1.42 ± 0.04 | 0.34 ± 0.12 |
| | 3.0 | 1.38 ± 0.06 | 0.19 ± 0.9* |
| Verb. II | 0.3 | 1.42 ± 0.07 | 0.33 ± 0.12 |
| | 3.0 | 1.38 ± 0.06 | 0.19 ± 0.9* |

Bei beiden Präparaten treten erst nach einer Dosis von 3 mg/kg signifikante systemische Wirkungen (*$p < 0.05$, Dunnett's-Test), gemessen als Hemmung des Pfotenödems, auf. Dies entspricht dem Verhalten von Prednicarbat.

2 b) Prüfung auf systemische Wirkung: Glukoneogenese an Ratten

Eine empflindliche Methode zum Nachweis systemischer Wirkung auf den Kohlhydratstoffwechsel ist die Prüfung der glukoneogenetischen Wirkung von Corticosteroiden an der adrenalektomierten Ratte. Drei Tage vor dem Versuch werden Gruppen zu je 6 Ratten in Pentobarbitalnarkose adrenalektomiert und mit 0.9 % Kochsalzlösung als Trinkflüssigkeit versorgt. Zwei Tage später, d.h. 24 Std. vor Versuchsbeginn, wird das Futter entzogen, um die Glykogenvorräte in der Leber zu reduzieren.

Am Versuchstag werden die Prüfpräparate subcutan appliziert, gelöst in Sesamöl (2 ml/kg). Sechs Stunden später werden die Tiere dekapitiert, die Leber wird entnommen und 1 g davon in 5 ml 0.6 molarer Perchlorsäure aufgenommen. Nach Homogenisierung wird die freie Glukose im Überstand des Zentrifugats bestimmt, das Zentrifugat (Glykogen) enzymatisch mit Amyloglukosidase aufgespalten und hierin ebenfalls der Glukosegehalt bestimmt (Hexokinase-Methode, Boehringer Mannheim). Es wurden die folgenden Ergebnisse erhalten:

| Behandlung | Dosis | Leberglykogen | Glykogen + Glukose |
|---|---|---|---|
| | (mg/kg s.c.) | | mg/100 g Leber |
| Kontrolle I | Sesamöl | 0.5 ± 0.3 | 8.9 ± 1.2 |
| Prednicarbat | 0.3 | 1.0 ± 1.7 | 19.3 ± 4.3 |
| Prednicarbat | 3 | 111 ± 46* | 170 ± 46* |
| Verbindung I | 0.3 | 0.5 ± 0.7 | 11.3 ± 2.0 |
| Verbindung I | 3 | 2.4 ± 2.4 | 25.9 ± 13 |
| Kontrolle II | Sesamöl | 2.4 ± 0.5 | 14.1 ± 3.6 |
| Verbindung II | 0.3 | 2.8 ± 1.4 | 23.6 ± 6.8 |
| Verbindung II | 3 | 7.4 ± 10 | 37.9 ± 30 |

Aus den aufgeführten Ergebnissen zur Glukose-/Glykogenneubildung ist zu entnehmen, daß die Verbindung I und die Verbindung II mit 3 mg/kg noch keine signifikante Wirkung haben, während Prednicarbat hier bereits eine geringe aber signifikante ($p < 0{,}05$, t-Test) Wirkung aufweist. Bei den Verbindungen I und II ist daher der therapeutische Vorteil (geringe systemische Wirkung) größer als bei Prednicarbat.

Beispiele:

Zu den im folgenden aufgeführten Beispielen sind die nachstehenden allgemeinen Bemerkungen zu machen:

Die Schmelzpunkte werden im Apparat nach Tottoli (Fa. Büchi) oder auf der Kofler-Heizbank der Fa. Reichert (Austria), Typ 7841, bestimmt und sind nicht korrigiert.
Die IR-Spektren (in KBr) werden mit dem Gitterspektrophotometer Perkin-Elmer 521 aufgenommen. Es werden jeweils nur die charakteristischen Banden angeführt. Die Aufnahme der UV-Spektren (in Methanol) erfolgte mit dem Spektralphotometer Beckmann DK 1 A. Die massenspektroskopischen Untersuchungen (MS) werden vorwiegend mit dem Gerät MS 9 (Fa. AEI) durchgeführt. Angabe der MS-Spektren (Molgewichtspeak) überwiegend in: MS = m/z = ... ($M + H^+$) (Messung mit Reinistopen), d. h. es wurde jeweils die monoisotopische Molmasse erfaßt. In der Regel wurden FAB-MS-Spektren gemessen.
Für die Dünnschicht-Chromatographie (DC) dienten Fertigplatten Kieselgel $F_{254}$ (Fa. Merck). Wenn nicht anders angegeben, wurde als Laufmittel Methylenchorid: Methanol = 19 : 1 benutzt (Laufstrecke 7 cm). Es wurde jeweils zweimal entwickelt. Die Flecken wurden entweder mit einer UV-Lampe bei 254 nm detektiert oder durch Besprühen mit 10 %-iger methanolischer Schwefelsäure sowie durch Erhitzen auf 100° C sichtbar gemacht. Die $R_F$-Werte sind immer nur relativ zu verstehen. Zur Säulenchromatographie wurde 15 Kieselgel 60, Korngröße 0.063 - 0.2 mm (Fa. Merck) verwendet.

Bei den Reaktionen mit Carbonsäurechloriden wird in vorteilhafter Weise oft absolutes Dioxan zum Reaktionsgemisch gegeben, z. B. bei Benzoytchlorid, wobei das Verhältnis Dioxan/Pyridin etwa 1:1 ist, und zur Reaktionsbeschleunigung wird das Reaktionsgemisch oft, insbesondere bei sterisch gehinderten oder weniger reaktiven Carbonsäurechloriden oder -anhydriden auf etwa 60°C erwärmt (DC-Verfolgung der Reaktionsverläufe).
Die Charakterisierung der Reaktionsprodukte kann durch Dünnschicht-Chromatograpie (DC) erfolgen; hierbei haben die Reaktionsprodukte $R_F$-Werte von etwa 0.65 - 0.75. In der Regel werden die Reaktionsprodukte durch Massenspektren mit MS = m/z = ... ($M + H^+$) charakterisiert (In der Regel FAB-Spektren); es wird jeweils die monoisotopische Molmasse erfaßt. Die $M + H^+$-Werte wurden jeweils aufgerundet. Auch IR-, $^1$H-NMR- und UV-Spektren können zur Charakterisierung herangezogen werden.

Beispiel 1

Prednisolon-17-ethylcarbonat-21-benzoat

a) Zu einer Lösung von 0.65 g (0.0015 Mol) Prednisolon-17-ethylcarbonat in 5 ml absolutem Aceton und 2 ml absolutem Pyridin werden bei 0° C und Rühren 0.23 ml Benzoesäurechlorid zugetropft. Man läßt 20 Std. bei Raumtemperatur (21°C) rühren, erwärmt, falls ein Dünnschicht-Chromatogramm noch Edukt detektiert, noch einige Stunden (ca. 5 Std.) auf 40-50°C, läßt abkühlen, gießt in 60 ml halbgesättigte wäßrige Kochsalzlösung ein,

dekantiert die wäßrige Phase von ausgefallenem Öl oder Wachs ab (falls ein Kristallisat ausfällt, filtriert man dieses ab), nimmt die Ausfällung in Methylenchlorid auf, wäscht die organische Phase mit Wasser, trocknet z.B. mit Natriumsulfat und destilliert vom Lösungsmittel ab. Der hinterbliebene Rückstand (0.8 g) wird in Ethanol und Methylenchlorid gelöst und anschließend durch Zugabe von Diethylether bzw. Diisopropylether umkristallisiert und ergibt 0.58 g Prednisolon-17-ethylcarbonat-21-benzoat vom Schmp. 133-135° (Zers.).

Im DC (Laufmittel: $CH_2Cl_2$:$CH_3OH$ = 19 : 1) zeigt dieses, wie auch die o. a. Ausfällung, neben dem Hauptfleck bei $R_F$ = ca. 0.7 noch Nebenflecke bei hauptsächlich $R_F$ etwa 0.5 - 0.55 und etwa 0.9. Zur Reindarstellung (DC) wird an Kieselgel [Korngröße 0.063 - 0.2000 mm (Merck AG), Säule 20 x 3 cm] mit Methylenchlorid/Methanol = 998 : 2 fraktioniert chromatographiert (50 ml-Fraktionen). Die Fraktionen, die im DC anschließend einen $R_F$ = Wert von etwa 0.7 zeigen, werden vereinigt. Nach Abdestillieren der Eluationsmittel erhält man aus Diethylether und/oder Ethanol, Methylenchlorid, Diethylether (oder Diisopropylether) 2.4 g (im besten Reproduktionsansatz 3.8 g) kristallisiertes Prednisolon-17-ethylcarbonat-21-benzoat vom Schmp. 136°C.

MS: m/z = 537 - ($M + H^+$)
DC: $R_F$ = ca. 0.7 (AP = 0.4) (AP = Ausgangsprodukt)

Auch die ölige bzw. wachsartige ölige o.a. Ausfällung zeigt nach dem Trocknen: MS: m/z = 537 ($M + H^+$)

b) Zu einer Lösung von 1.3 g Prednisolon-17-ethylcarbonat in 12 ml Pyridin werden bei 0°C und Rühren 0.45 ml Benzoylchlorid in 10 ml absolutem Dioxan zugetropft, und es wird 48 Std. bei 21°C gerührt (und ggf. noch 4 - 6 Std. bei 40° - 60°C gerührt, bis DC kein AP mehr zeigt). Nach analoger Aufarbeitung, Isolierung und gegebenenfalls Reindarstellung wie unter Beispiel 1 a) angegeben erhält man nach dem Kristallisieren aus Diethylether 1.1 g Prednisolon-17-ethylcarbonat-21-benzoat, das hinsichtlich Schmp., MS und DC identisch mit dem Reaktionsprodukt nach Beispiel 1 a) ist. Wird obiges Reaktionsgemisch bei 0°-5°C gerührt, so sind oft längere Reaktionszeiten als oben angegeben, erforderlich.

c) Setzt man in Beispiel 1 b) anstelle von Benzoylchlorid 0.6 ml Benzoesäureanhydrid ein und führt die Reaktion sowie Aufarbeitung und Reindarstellung in gleicher Weise, wie unter Beispiel 1 b) bzw. 1 a) beschrieben, durch, so erhält man das gleiche Reaktionsprodukt mit den gleichen Daten wie unter Beispiel 1 b) bzw. 1 a) beschrieben.

Beispiel 2:

Prednisolon-17-ethylcarbonat-21-phenylacetat

a) In gleicher Weise, wie unter Beispiel 1 a) angegeben, werden 0.65 g Prednisolon-17-ethylcarbonat umgesetzt, aufgearbeitet und chromatographisch gereinigt. Anstelle des dort eingesetzten Benzoylchlorids werden aber 0.23 g Phenylessigsäurechlorid eingesetzt. Nach Umkristallisation aus tert.-Butanol/Diisoproylether werden 0.44 g Prednisolon-17-alkylcarbonat-21-phenylacetat vom Schmp. ca. 180°C erhalten. Neben dem Hauptfleck bei $R_F$ = 0.8 werden noch mehrere meist schwache Nebenflecke unter- und oberhalb $R_F$ = 0.8 detektiert.

MS: m/z = 551 ($M+H^+$)
DC: $R_F$ = 0.80 ($R_F$(ED) = 0.45)

ED = Edukt

b) Zu einer Lösung von 1.1 g (0.0025 mol) Prednisolon-17-ethylcarbonat und 1.2 g (0.0088 mol) Phenylessigsäure (5 Std. in Vakuum über $P_2O_5$ bei ca. 50 - 60°C getrocknet) in 6 ml absolutem Pyridin gibt man unter Rühren und bei 20°C eine frisch zubereitete Mischung von 26 mg konzentrierter Schwefelsäure in 2.5 ml absolutem Pyridin (Suspension von Pyridinium-Sulfat). Nach 15 Min. Rühren gibt man 720 mg (0.0035 mol) N,N'-Dicyclohexylcarbodiimid zu. Aus der anfänglichen klaren Lösung fällt bald ein kristalliner Niederschlag von gebildeten N,N'-Dicyclohexylharnstoff aus. Man rührt solange bis im DC kein Edukt mehr nachweisbar ist und das Reaktionsprodukt bei $R_F$ = 0.8 detektierbar ist (in der Regel 16 Stunden Reaktionszeit; eine längere Reaktionszeit, z. B. Stehen bzw. Rühren übers Wochenende beeinträchtigt nicht das Reaktionsergebnis). Hiernach gibt man 0.3 ml Essigsäure hinzu und läßt den Ansatz noch 1 Std. bei 20°C 24 - 48 Std. im Tiefkühlschrank (ca. -15°C) stehen. Man filtriert vom ausgefallenem N,N'-Dicyclohexylharnstoff ab, wäscht diesen mit etwa -15°C kaltem Pyridin und rührt das Filtrat in ca. 400 ml viertelgesättigte wäßrige Kochsalzlösung ein, gibt etwa 5 ml Ethanol hinzu, filtriert die öligkristalline Ausfällung ab, wäscht diese mehrmals mit Wasser und nimmt diese mit etwa 20 ml Methylenchlorid auf. Nach dem Trocknen mit Natriumsulfat destilliert man ab und kristallisiert den Rückstand durch Zugabe von Diethylether oder Diisopropylether.
Man erhält 1,1 g Prednisolon-17-ethylcarbonat-21-phenylacetat vom Schmp. 179 - 183°C, das aus tert.-Butanol/Diethylether umkristallisiert werden kann. Schmp.: 184- 185°C

MS: m/z = 551 (M + $H^+$)
DC: $R_F$ = 0.80 ($R_F$ v. ED = 0.45)

Eine chromatographische Reinigung wie unter Beispiel 2 a) angegeben, ist hier zur Reindarstellung nicht erforderlich.

c) Es wird ein weiterer analoger Ansatz, wie unter Beispiel 2 b) beschrieben durchgeführt; allerdings wird der saure Katalysator, konzentrierte Schwefelsäure in Pyridin, weggelassen. Nach etwa 5-facher Reaktionszeit, wie unter Beispiel 2 b) angegeben, zeigt eine DC-Probe kein Edukt mehr. Nach analoger Aufarbeitung und Reindarstellung, wie unter Beispiel 2 b) angegeben, werden 0.9 g Prednisolon-17-ethylcarbonat-21-phenylacetat mit den gleichen Kenndaten, wie unter Beispiel 2 b) angegeben, erhalten.

Wird anstelle von Pyridin absolutes Dimethylformamid als Lösungsmittel verwendet, so erhält man die Titelverbindung ebenfalls mit den gleichen Daten.

d) Es wird ein weiterer analoger Ansatz, wie unter Beispiel 2 b) beschrieben durchgeführt. Anstelle der Schwefelsäure werden aber 60 mg p-Toluolsulfonsäure zugegeben. Nach analoger Aufarbeitung und Reindarstellung, wie unter Beispiel 2 b) angegeben, werden 1,2 g Prednisolon-17-ethylcarbonat-21-phenylacetat mit den gleichen Kenndaten, wie in Beispiel 2 b) angegeben, erhalten.

Beispiel 3

Prednisolon-17-ethylcarbonat-21-(3)-phenylpropionat

Zu einer Lösung von 10 g Prednisolon-17-ethylcarbonat in 84 ml absolutem Aceton und 32 ml absolutem Pyridin werden bei 0°C und Rühren 4.15 g 3-Phenylpropionsäurechlorid zugetropft, und es wird 6 Stunden unter Rückfluß gerührt. Eine DC-Probe zeigt noch keine vollständige Umsetzung. Daher werden weitere 2.5 g 3-Phenylpropionsäurechlorid sowie 30 ml Pyridin zugetropft und weitere 6 Std. bei Rückfluß gerührt (DC-Probe zeigt vollständige Umsetzung). Man rührt in 1 l kochsalzhaltiges Wasser ein, nimmt das ausgefallene Öl in Essigester auf, wäscht mit Wasser, trocknet und destilliert das Lösungsmittel ab. Zur Reindarstellung wird an 200 g Kieselgel (Korngröße 70 - 200 µm), säulenchromatographiert (Eluationsmittel Essigester). Die DC-einheitlichen Fraktionen werden vereinigt, das Eluationsmittel wird abdestilliert und der Rückstand aus Diethylether zur Kristallisation gebracht. Man erhält 9.0 g der o. a. Titelverbindung.
Schmp.: 146 - 147°C

MS: m/z = 565 (M + $H^+$)
DC: $R_F$ = 0.7

Beispiel 4

Prednisolon-17-ethylcarbonat-21-(thienyl-2-essigsäure)ester

In abs. Aceton/Pyridin werden 0.6 g Prednisolon-17-ethylcarbonat mit 0.21 g 2-Thienylacetylchlorid umgesetzt, aufgearbeitet und säulenchromatographisch rein dargestellt. Aus Diethylether erhält man 340 mg der o.a. Titelverbindung. Schmp. 195 - 198°C

MS: m/z = 557 (M + $H^+$)
DC: $R_F$ = 0.7

Beispiel 5

Prednisolon-17-ethylcarbonat-21-(thiophen-2-carbonsäure)ester

In absolutem Pyridin Aceton werden 0.6 g Prednisolon-17-ethylcarbonat mit 0.21 g Thiophen-2-carbonsäurechlorid umgesetzt; es wird aufgearbeitet, und das Produkt wird säulenchromatographisch rein dargestellt. Aus Diethylether erhält man 440 mg der o.a. Titelverbindung. Schmp. 142°C

MS: m/z = 543 ($M + H^+$); DC: $R_F$ = 0.7

Beispiel 6

Prednisolon-17-ethylcarbonat-21-(furan-2-carbonsäureester)

In absolutem Pyridin Aceton werden 0.6 g Prednisolon-17-ethylcarbonat mit 0.21 g Furan-2-carbonsäurechlorid anstelle des Zimtsäurechlorids umgesetzt; es wird aufgearbeitet, und das Produkt wird säulenchromatographisch rein dargestellt. Aus Diethylether erhält man 540 mg der o.a. Titelverbindung. Schmp. 170°C (bei einem anderem Ansatz war
Fp = 214°C, Doppelschmelzpunkt)

MS: m/z = 527 ($M + H^+$)
DC: $R_F$ = 0.7

Beispiel 7

Prednisolon-17-ethylcarbonat-21 -phenylacetat

408 mg (0,003 mol) wasserfreie Phenylessigsäure und 492 mg (0,003 mol) N, N'-Carbonyldi(1H-1,2,4-triazol) oder eine äquimolare Menge N,N'-Carbonyldiimidazol, werden 1 Std. in 15 ml absolutem Pyridin bei 20°C, gerührt. Hiernach werden 1,43 g (0,0033 mol) getrocknetes Prednisolon-17-ethylcarbonat in 10 ml absolutem Pyridin zugetropft. Nach Zugabe einer katalytischen Menge (5 mg) Natriumhydrid wird übers Wochenende bei 20°C gerührt und über Nacht bei 20°C stehen gelassen. Nach Abfiltrieren eines gebildeten Niederschlags wird das Filtrat in 200 ml kochsalzhaltiges Wasser + 10 ml Ethanol eingerührt. Durch Abdekantieren wird der ausgefallene ölige Niederschlag in Methylenchlorid gelöst und an Kieselgel analog Beispiel 1a) chromatographisch aufgetrennt. Das erhaltene Kristallisat aus tert. Butanol/Diethylether zeigt die gleichen Kenndaten wie das Reaktionsprodukt gemäß Beispiel 2 a) und stellt daher Prednisolon-17-ethylcarbonat-21-phenylacetat dar.

Beispiel 8

Prednisolon-17-n-propylcarbonat-21-(3)-phenylpropionat

Zu einer Lösung von 340 mg Prednisolon-17-n-propylcarbonat in 3 ml absolutem Pyridin wird bei 0°C und unter Rühren eine Lösung von 300 mg 3-Phenyl-propionsäurechlorid in 1 ml absolutem Dioxan zugetropft. Nach 5 bis 6 Std. Rühren bei 0°C (DC zeigt vollständige Bildung des gewünschten Reaktionsproduktes) gießt man in 100 ml halbgesättigte wässrige Kochsalzlösung ein, isoliert die Ausfällung (ölig oder Wachs) über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert mit Diisopropylether oder Diethylether oder Petrolether, filtriert ab und kristallisiert (ggf.) aus Ethanol/Ether (ggf. Zusatz von Petrolether) um. Man erhält 405 mg der o.a. Titelverbindung vom Schm.: 128-134°C

MS: m/z = 579 ($M + H^+$)
DC: $R_F \cong 0,7$

Beispiel 9

Prednisolon-17-n-propylcarbonat-21-phenylacetat

Werden, wie in Beispiel 2 b) beschrieben, 1,1 g Prednisolon-17-n-propylcarbonat mit 1,2 g Phenylessigsäure und 720 mg N,N'-Dicyclohexylcarbodiimid sowie Pyridiniumsulfat umgesetzt, so erhält man 1,0 g der o.a. Titelverbindung
(Smp.: 178°C).

MS: m/z = 565 (M + H+)
DC: $R_F \cong 0{,}8$

Beispiel 10

Prednisolon-17-iso-propylcarbonat-21-phenylacetat

Wird anstelle des in Beispiel 9 genannten Corticoids 1,1 g Prednisolon-17-Isopropylcarbonat mit Phenylessigsäure umgesetzt, so erhält man 1,1 g der o.a. Titelverbindung. Fp = 200-202°C

MS: m/z = 565 (M + H+)
DC: $R_F \cong 0{,}8$

Beispiel 11

Prednisolon-17-n-butylcarbonat-21-phenylacetat

Wird anstelle des in Beispiel 9 genannten Corticoids 1,15 g Prednisolon-17-n-Butylcarbonat mit Phenylessigsäure umgesetzt, so erhält man 1,2 g der o.a. Titelverbindung. Fp: 198°C

MS: m/z = 579 (M+H+)
DC: $R_F \cong 0{,}8$

Beispiel 12

Betamethason-17-ethylcarbonat-21-(3)-phenyl-propionat

In gleicher Weise, wie unter Beispiel 8 beschrieben, werden 340 g Betamethason-17-ethylcarbonat mit 300 mg 3-Phenylpropionsäurechlorid umgesetzt. Man erhält 380 mg der o.a. Titelverbindung vom Schmp. ~167°C

MS: m/z = 597 (M + H+)
DC: $R_F \cong 0{,}8$

Beispiel 13

Betamethason-17-ethylcarbonat-21-phenylacetat

a.) In gleicher Weise, wie unter Beispiel 8 beschrieben, werden 340 mg Betamethason-17-ethylcarbonat mit 300 mg Phenylacetylchlorid anstelle des 3-Phenylpropionchlorids umgesetzt. Man erhält 230 mg der o.a. Titelverbindung vom Schmp. 168°C

MS: m/z = 583(M + H+)
DC: $R_F \cong 0{,}7$

b.) Werden, wie in Beispiel 2 b) beschrieben, 1,1 g Betamethason-17-ethylcarbonat mit 1,2 g Phenylessigsäure und 720 mg N,N'-Dicyclohexylcarbodiimid sowie Pyridiniumsulfat umgesetzt und aufgearbeitet, so erhält man 1,1 g der o.a. Titelverbindung mit den gleichen unter Beispiel 25a) angegebenen physikalischen und spektralen Daten.

Beispiel 14

Betamethason-17-ethylcarbonat-21-(thienyl-2-essigsäure)ester

In ähnlicher Weise werden 0,3 g Betamethason-17-ethylcarbonat in absolutem Pyridin mit 0.21 g 2-Thienylacetylchlorid umgesetzt und aufgearbeitet. Aus Diethylether erhält man 400 mg der o.a. Titelverbindung. Schmp. 153-159°C (unscharf)

MS: m/z = 589 (M + $H^+$)
DC: $R_F$ = 0.7

Beispiel 15

Betamethason-17-ethylcarbonat-21-(furan-2-carbonsäureester)

In absolutem Pyridin Aceton werden 0,3 g Betamethason-17-ethylcarbonat mit 0.21 g Furan-2-carbonsäurechlorid anstelle des Zimtsäurechlorids umgesetzt und aufgearbeitet. Aus Diethylether erhält man 540 mg der o.a. Titelverbindung. Schmp. ~170°C (unscharf)

MS: m/z = 559 (M + $H^+$)
DC: $R_F$ = 0.7

Beispiel 16

Betamethason-17-ethylcarbonat-21-(indol-3-essigsäure)ester

In abs. Aceton/Pyridin werden 1,1 g Betamethason-17-ethylcarbonat mit 1,2 g Indolyl-3-essigsäure und 720 mg N,N'-Dicyclohexylcarbodiimid sowie Pyridiniumsulfat umgesetzt und aufgearbeitet. Man erhält 0,95 g der o.a. Titelverbindung als Wachs, die nicht chromatographisch rein dargestellt wurde.

MS: m/z = 622 (M + $H^+$)
DC: $R_{F \cong}$ 0,65 (Hf)

Beispiel 17

Prednisolon-17-ethylcarbonat-21-(indol-3-essigsäure)ester

Zu einer Lösung von 2,2 g Prednisolon-17-ethylcarbonat und 3,1 g 3-Indolessigsäure (getrocknet) in 15 ml absolutem Pyridin gibt man unter Rühren und bei 20° Pyridiniumsulfat (aus 56 mg conz. Schwefelsäure in 2,5 ml absol. Pyridin, gemäß Beispiel 2b). Nach 30 Minuten Rühren (20°C) gibt man 1,55 g N,N'-Dicyclohexylcarbodiimid hinzu. Nach 48 Stunden Rühren bei 20°C zeigt das Massenspektrum m/z = 590,4 (M+$H^+$) und kein m/z = 433 (M + $H^+$) für das Ausgangssteroid. Nach analoger Weiterbehandlung und Aufarbeitung wie unter Beispiel 2b) erhält man nach dem Eingießen in ca. 500 ml halbgesättigte Kochsalzlösung eine ölige Ausfällung, die in ein Wachs übergeht. Man dekantiert bzw. filtriert das Wachs ab, wäscht es mit Wasser und trocknet es im Exsikkator im Vakuum über $P_2O_5$. Nach dem Anreiben mit Petrolether erhält man 1,55 g der Titelverbindung als amorphes Produkt.
MS (von Wachs bzw. amorphen Material): m/z = 590 (M+$H^+$) DC ≅ 0,75 (Hauptfleck = Hf + wenige schwache Nebenflecke). Zur Reinstdarstellung wird mit Methylenchlorid/Methanol = 99,5:0,5 an Kieselgel chromatographiert (Säule: Durchmesser = 5 cm; h = 20 cm). Die mit $R_{F \cong}$ 0,75 anfallenden Eluat-Fraktionen werden vereinigt und von den Lösungsmitteln durch Destillation befreit. Der Rückstand wird aus Diethylether zur Kristallisation gebracht. Man erhält 1,2 g der Titelverbindung vom Schmp. 145°C mit den gleichen Daten für MS und DC wie die wachsartige bzw. amorphe Titelverbindung.

Beispiel 18

Dexamethason-17-ethylcarbonat-21-phenylacetat

a.) Werden, wie in Beispiel 17) beschrieben, 0,55 g Dexamethason-17-ethylcarbonat mit 0,6 g Phenylessigsäure anstelle der 3-Indolylessigsäure, und 360 mg N,N'-Dicyclohexylcarbodiimid sowie 15 mg konzentrierte Schwefelsäure in 1,25 ml Pyridin (= Pyridiniumsulfat) in insgesamt 4,5 ml absolutem Pyridin bei Raumtemperatur umgesetzt, aufgearbeitet als Wachs oder amorph isoliert und (gegebenenfalls) durch Chromatographie kristallisiert rein dargestellt, so erhält man 540 mg Dexamethason-17-ethylcarbonat-21-phenylacetat, Schmp.: 185-189°C.

MS: m/z = 583 (M + $H^+$) (kristallisiert, als Wachs oder amorph)
DC:

$R_F \cong 0,7$ }

In gleicher Weise, wie in Beispiel 18 a) beschrieben, werden ausgehend von (anstelle von Dexamethason-17-ethylcarbonat)

b.) Hydrocortison-17-ethylcarbonat das Hydrocortison-17-ethylcarbonat-21-phenylacetat (Ms: m/z = 553 (M + $H^+$); $R_F \cong 0,8$)
c.) Cortison-17-ethylcarbonat das Cortison-17-ethylcarbonat-21-phenylacetat ($R_F \cong 0,8$)
d.) 6α-Methylprednisolon-17-ethylcarbonat das 6α-Methylprednisolon-17-ethylcarbonat-21-phenylacetat ($R_F \cong 0,75$)
(MS: m/z = 565 (M + $H^+$)
e.) Prednison-17-ethylcarbonat das Prednison-17-ethylcarbonat-21-phenylacetat ($R_F \cong 0,7$)
f.) 6α-Fluor-prednisolon-17-ethylcarbonat das 6α-Fluor-prednisolon-17-ethylcarbonat-21-phenylacetat ($R_F \cong 0,8$)
(MS: m/z = 569 (M + $H^+$)
g.) 6α-Fluor-dexamethason-17-ethylcarbonat das 6α-Fluor-dexamethason-17-ethylcarbonat-21-phenylacetat ($R_F \cong 0,8$; MS: m/z 601 (M + H +))
h.) 6α-Fluor-betamethason-17-ethylcarbonat das 6α-Fluor-betamethason-17-ethylcarbonat-21-phenylacetat ($R_F \cong 0,75$)
i.) 6α,16α-Dimethyl-prednisolon-17-ethylcarbonat das 6α,16α-Dimethyl-prednisolon-17-ethylcarbonat-21-phenylacetat ($R_F \cong 0,75$)
j.) vom 17α-Ethylcarbonat von Reichsteins Substanz S das 17α-Ethylcarbonat, 21-phenylacetat von Reichsteins Substanz S ($R_F \cong 0,85$; MS: m/z = 537 (M + $H^+$))
k.) Beclomethason-17α-ethylcarbonat das Beclomethason-17α-ethylcarbonat-21-phenylacetat ($R_F = \cong 0,8$)
l.) 6α-Methyl-9α-fluor-prednisolon-17-ethylcarbonat das 6α-Methyl-9α-fluor-prednisolon-17-ethylcarbonat-21-phenylacetat ($R_F \cong 0,85$; MS: m/z = 583 (M + $H^+$))
m.) Betamethason-17-n-propylcarbonat das Betamethason-17-n-propylcarbonat-21-phenylacetat ($R_F = \cong 0,8$)
n.) Dexamethason-17-iso-propylcarbonat das Dexamethason-1 7-iso-propylcarbonat-21-phenylacetat ($R_F = \cong 0,75$)
o.) Prednisolon-17-n-propylcarbonat das Prednisolon-17-n-propylcarbonat-21-phenylacetat ($R_F = \cong 0,8$)
p.) Prednisolon-17-iso-propylcarbonat das Prednisolon-17-iso-propylcarbonat-21-phenylacetat ($R_F = \cong 0,8$)
q.) Prednisolon-17-n-butylcarbonat das Prednisolon-17-n-butylcarbonat-21-phenylacetat ($R_F = \cong 0,8$)
r.) Prednisolon-17-iso-butylcarbonat das Prednisolon-17-iso-butylcarbonat-21-phenylacetat ($R_F = \cong 0,7$)
s.) Prednisolon-17-methoxyethylcarbonat das Prednisolon-17-methoxyethylcarbonat-21-phenylacetat ($R_F = \cong 0,8$)

als Öl oder Wachs oder amorph oder kristallisiert erhalten.

Beispiel 19

Prednisolon-17-ethylcarbonat-21-phenylacetat

a) Zu einer Lösung von 2,10 g Prednisolon-17-ethylcarbonat und 1,20 g Phenylessigsäure in 40 ml absol. Methylenchlorid gibt man bei 0°C und Rühren 120 mg 4-Dimethylaminopyridin und 1,75 g Dicyclohexylcarbodiimid. Die zunächst klare Reaktionslösung trübt sich bald. Nach ca. 36 Stunden Rühren bei Zimmertemperatur zeigt eine DC-Probe kein Edukt mehr. Man bewahrt dann 2 Tage bei -15°C (Tiefkühlschrank) auf, filtriert den ausgefallenen Dicyclohexylharnstoff ab, wäscht diesen mit etwas -15°C kaltem Methylenchlorid und zieht das organische Lösungsmittel im Vakuum ab. Der hinterbliebene Rückstand wird aus siedendem Diethylether zur Kristallisation gebracht und aus Ethanol/Diethylether umkristallisiert. Man erhält 1,9 g der o.a. Titelverbindung (strahlend weiße Kristalle) mit den gleichen unter Beispiel 2b) angegebenen Daten (MS, DC, Schmelzpunkt. Der Schmelzpunkt ist gegenüber Beispiel 2b) um etwa 2° höher: Schmp. 166-168°C,

b) bei einem analogen Ansatz gemäß Beispiel 29 a wird das Methylenchlorid durch Dimethylformamid als Lösungsmittel ersetzt. Ansonsten wird genau wie unter Beispiel 30a) angegeben verfahren. Nach der Aufarbeitung erhält man 1,7 g der o.a. Titelverbindung mit Schmp.: 165-167°C

Beispiel 20

Prednisolon-17-ethylcarbonat-21-phenylacetat

a) Eine Mischung von 216 mg Prednisolon-17-ethylcarbonat oder 270 mg 21-(tert.-Butyldimethylsiloxy-prednisolon-17-ethylcarbonat, 136 mg Phenylessigsäure, 210 mg Trifluoressigsäureanhydrid sowie 6 mg wasserfreie p-Toluolsulfonsäure werden 7 Stunden in 40 ml absol. Toluol oder Benzol unter Rückfluß gekocht. Hiernach wird in 6 %ige wäßrige Natriumbicarbonatlösung eingegossen und intensiv durchgerührt. Man wäscht mit Wasser, trocknet, zieht das Lösungsmittel ab und chromatographiert an Kieselgel (s. Beispiel 2b). Das bei DC = $R_F \cong 0{,}7$ laufende Produkt wird aus Diethylether kristallisiert. Es ist in allen Daten mit dem unter Beispiel 2 angegebenen Reaktionsprodukt identisch.

b) Bei einem weiteren Ansatz werden 700 mg Prednisolon-17-ethylcarbonat in 20 ml absolutem Dioxan mit 1,5 g Phenylessigsäure und 0,75 ml Trifluoressigsäureanhydrid versetzt. Nach 30 Stunden Rühren bei 20°C rührte man in 40 ml Wasser, das 2 g Natriumbicarbonat enthält, ein. Das erhaltene wachsartige Produkt wird nach dem Trocknen wie unter Beispiel 30 a) oder 2 b) chromatographiert und aus Diethylether kristallisiert. Man erhält o.a. Titelverbindung mit den gleichen, wie unter Beispiel 2, angegebenen Daten.

Beispiel 21

Prednisolon-17-ethylcarbonat-21-phenylacetat

Eine Lösung von 286 mg Phenylessigsäure in 14 ml absol. Methylenchlorid wurde bei 0°C und unter Rühren nacheinander mit 435 mg N,N'-Dicyclohexylcarbodiimid, 43 mg N,N'-Dimethylaminopyridin sowie 700 mg Prednisolon-17-ethylcarbonat versetzt. Nach 18 Stunden Rühren bei 20°C wird mit 40 ml gesättigter wäßriger Natriumhydrogencarbonatlösung, mit 30 ml wäßriger Salzsäure (2 mol $dm^{-3}$) sowie Wasser gewaschen. Die Methylenchloridphase wird im Vakuum im Rotationsverdampfer eingedampft, und der Rückstand aus Ethanol/Methylenchlorid/ Diethylether kristallisiert. Man erhält 520 mg der o.a. Titelverbindung, die in allen Daten mit dem nach Beispiel 2a oder b erhaltenem Produkt identisch ist.

Beispiel 22

Prednisolon-17-ethylcarbonat-21-phenylacetat

150 mg Phenylessigsäure und 432 mg Prednisolon-17-ethylcarbonat werden in 3 ml abs. Methylenchlorid sowie 5 ml abs. Pyridin gelöst und mit 0,25 ml 50 %iger Propanphosphonsäureanhydridlösung in abs. Methylenchlorid sowie 10 mg 4-Dimethylaminopyridin versetzt. Nach 8 Stunden Rühren bei ca. 40°C (Ölbad) wird in Eiswasser, das zur Neutralisation Natriumbicarbonat enthält, eingegossen. Es wird mit Essigester extrahiert, mit wäßriger $KHSO_4$-Lösung sowie Wasser gewaschen. Nach dem Abdestillieren wird der Rückstand analog Beispiel 1 a) chromatographiert. Neben Ausgangsmaterial und Prednisolon enthält eine Eluatfraktion auch die gewünschte o.a. Titelverbindung mit den gleichen Daten, wie unter Beispiel 2 b angegeben.

Beispiel 23

Prednisolon-17-ethylcarbonat-21-phenylacetat

Zu einer Lösung von 226 mg Prednisolon-17-ethylcarbonat in 2 ml absolutem Pyridin wird bei 0°C und unter Rühren eine Lösung von 400 ml Phenylessigsäureanhydrid in 1 ml absolutem Dioxan zugetropft. Nach 5 bis 6 Stunden Rühren bei 0°C und 16 Stunden Rühren bei 20°C gießt man in 100 ml halbgesättigte wäßrige Kochsalzlösung ein, isoliert die wachsartige Ausfällung über ein Faltenfilter, nimmt diese mit Methylenchlorid (oder Essigester) auf, wäscht mit Wasser, trocknet mit Natriumsulfat, destilliert im Vakuum das Lösungsmittel ab, kristallisiert mit Diisopropylether oder Diethylether oder Petrolether, filtriert ab und kristallisiert aus Ethanol/Ether (ggf. Zusatz von Petrolether) um. Man erhält 145 mg der o.a. Titelverbindung vom Schmp.: 183°C

MS: m/z = 551 ($M + H^+$)
DC: $R_F \cong 0{,}7$

Beispiel 24

Prednisolon-17-ethylcarbonat-21-phenylcarbonat

Zu einer Lösung von 2,26 g Prednisolon-17-ethylcarbonat in 9 ml absol. Pyridin wird bei 0°C und unter Rühren eine Lösung von 4 ml Chlorameisensäure-phenylester in 12 ml absol. Dioxan zugetropft, wobei eine ölige Ausfällung auftrat. Nach 7 Stunden Rühren bei 0°C gießt man in 200 ml halbgesättigte Kochsalzlösung (aq) ein, filtriert das ausgefallene Öl über ein Faltenfilter ab, nimmt es mit Methylenchlorid auf und chromatographiert den Rückstand an Kieselgel (35-70 µm) mit Methylenchlorid/Methanol = 99,5:0,5. Die Fraktionen bei $R_F \cong 0,75$ werden vereinigt und aus Diisopropylether zur Kristallisation gebracht. Man erhält 1,1 g der o.a. Titelverbindung vom Schmp.: 114°C.

MS: m/z = 553 ($M + H^+$) DC: $R_F \cong 0,7$

Beispiel 25

Prednisolon-17-ethylcarbonat-21-(9-fluorenylmethyl)carbonat

In gleicher Weise, wie unter Beispiel 24 beschrieben, werden 2,26 g Prednisolon-17-ethylcarbonat mit 7,5 g Chlorameisensäure-(9-fluorenylmethyl)ester umgesetzt, aufgearbeitet und dargestellt. Man erhält 1,7 g der o.a. Titelverbindung als amorphes Produkt (aus Petrolether).

MS: m/z = 655 ($M + H^+$)
DC: $R_F$ - 0,7

Beispiel 26

Prednisolon-17-ethylcarbonat-21-phenylacetat

a) Eine Lösung von 510 mg Prednisolon-17-ethylcarbonat-21-mesylat (oder eine äquimolare Menge des analogen -21-p-chlor-benzolsulfonats), 145 mg Phenylessigsäure und 112 mg Triethylamin (hierbei findet intermediär Bildung des Triethylammoniumphenylacetats statt) in 25 ml Dimethylformamid (oder Acetonitril) werden 3 Stunden bei ca. 45°C (Ölbad) gerührt. Hiernach wird das Dimethylformamid bzw. Acetonitril im Vakuum abdestilliert und der Rückstand mit 30 ml Methylenchlorid behandelt. Man wäscht die organische Phase hintereinander mit 1 N wäßriger Salzsäure und 4 mal mit Wasser. Nach Chromatographie gemäß Beispiel 2 b und Kristallisation aus Diethylether erhält man die o.a. Titelverbindung mit den gleichen Daten, wie unter Beispiel 2b angegeben.

b) Zur gleichen Titelverbindung gelangt man, wenn 600 mg Prednisolon-17-ethylcarbonat-21-desoxy-21-jodid, 150 mg Phenylessigsäure, 2,5 ml Triethylamin in 25 ml Acetonitril 45 Minuten unter Rückfluß kocht und wie unter a) aufgearbeitet und isoliert.

c) 600 mg Prednisolon-17-ethylcarbonat-21-desoxy-21-jodid werden mit 200 ml Kalium-phenylacetat (Rhone-Poulenc) in 25 ml absol. Dimethylformamid 40 Minuten auf 100°C (Ölbad) unter Rühren erhitzt. hiernach kühlt man ab und gießt in halbgesättigte wäßrige Kochsatzlösung ein, wobei ein abfiltrierbares öliges Wachs ausfällt, das nach dem Abfiltrieren, Waschen mit Wasser und Trocknen (Vakuum über $P_2O_5$) an Kieselgel und nach dem Kristallisieren die o.a. Titelverbindung mit den gleichen Daten wie in Beispiel 2a bzw. 2b ergibt.

Analog zu diesen Beispielen sind die folgenden Beispiele von Tabellen 1 und 2, wobei R(1)' die gesamte Seitenkette an der $21CH_2O$-Gruppe darstellt.

Zur Charakterisierung der Syntheseprodukte wurden jeweils lediglich die nach den Massenspektren erhaltenen Molgewichtspeaks (m/z = .... ($M + H^+$) ausgewertet (als Öl oder Wachs oder amorph oder kristallisiert), und in der Regel erfolgte hiernach keine Reinstdarstellung durch Kristallisation (Umkristallisation) bzw. Chromatographie.

Tabelle 1:

Basis-Corticoid: Prednisolon

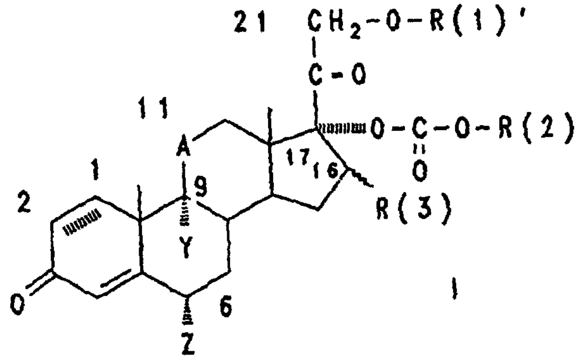

I

1,2-Pos. = △

A = 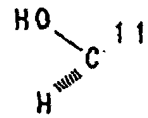

R(3) = H

Y = H

Z = H

△ = 1,2 Doppelb.

| Lauf. Nr. | eingesetzte(s) Carbonsäure Carbonsäure-chlorid Carbonsäure-anhydrid | Verfahrensvariante gem. Beispiel | R(2) | R(1)' | MS (m/z) (M+H+) |
|---|---|---|---|---|---|
| 1.1 | 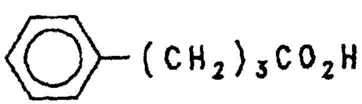 | 2b, | $-C_2H_5$ | 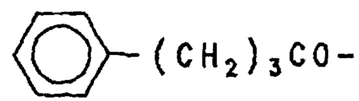 | 579 |
| 1.2 | 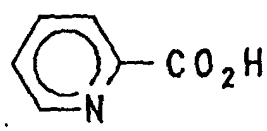 | 2b | $-C_2H_5$ | 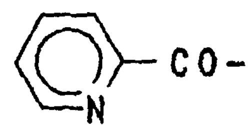 | 538 |

| Lauf. Nr. | eingesetzte(s) Carbonsäure Carbonsäure-chlorid Carbonsäure-anhydrid | Verfahrens-variante gem. Beispiel | R(2) | R(1)' | MS (m/z) (M+H⁺) |
|---|---|---|---|---|---|
| 1.3 | 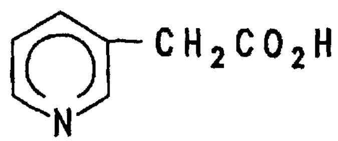 | 2b | $-C_2H_5$ | 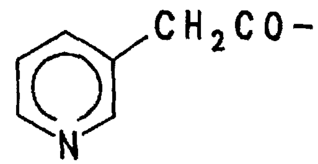 | 552 |
| 1.4 | 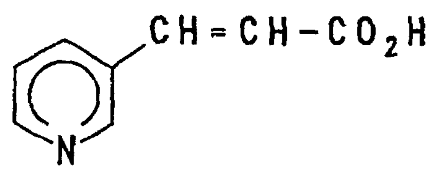 | 2b | $-C_2H_5$ | 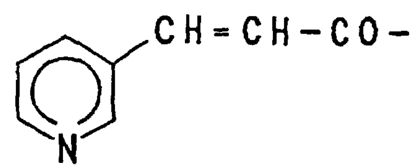 | 564 |

Tabelle 2:

Basis-Corticoid: Prednisolon

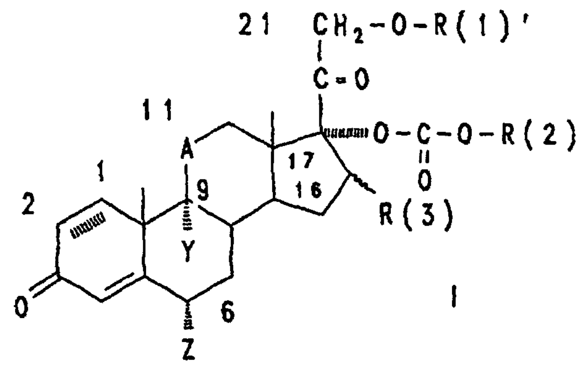

I

1,2-Pos. = △

A = 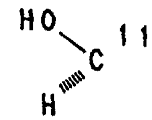

R(3) = H

Y = H

Z = H

△ = 1,2 Doppelb.

| Lauf. Nr. | eingesetzte(s) Carbonsäure Carbonsäure-chlorid Carbonsäure-anhydrid | Verfahrens-variante gem. Beispiel | R(2) | R(1)' | MS (m/z) (M+H⁺) |
|---|---|---|---|---|---|
| 2.1 | 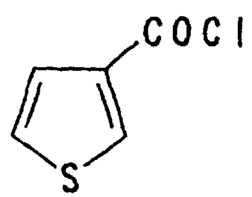 | 1a | $-C_2H_5$ | 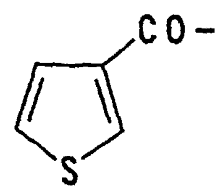 | 543 |

| | | | | | |
|---|---|---|---|---|---|
| 2.2 | 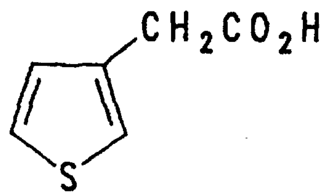 | 2b | $-C_2H_5$ | 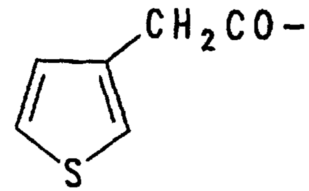 | 557 |
| 2.3 | 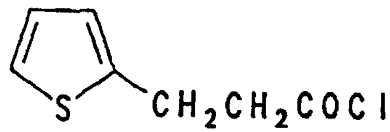 | 1a | $-C_2H_5$ | 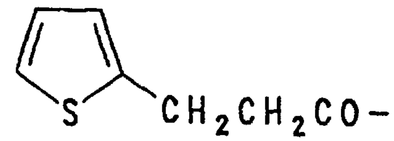 | 571 |
| 2.4 | 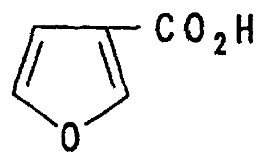 | 2b, | $-C_2H_5$ | 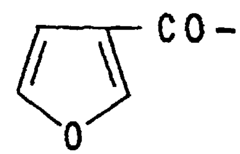 | 527 |
| 2.5 | 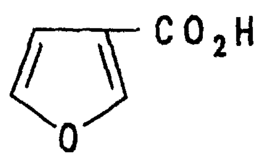 | 2b | $n-C_3H_7$ | 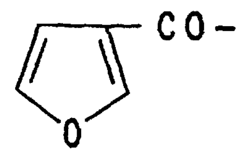 | 541 |

| | | | | | |
|---|---|---|---|---|---|
| 2.6 | 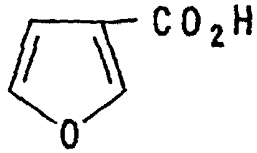 | 2b | n-$C_4H_9$ | 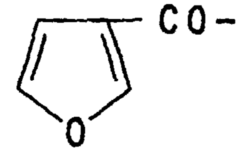 | 555 |
| 2.7 | 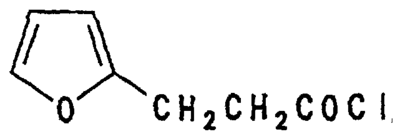 | 1a | -$C_2H_5$ | 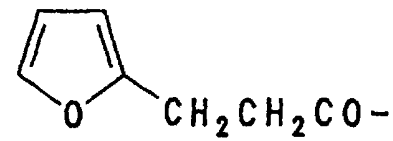 | 555 |
| 2.8 | 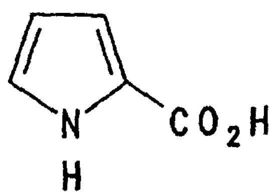 | 2b | -$C_2H_5$ | 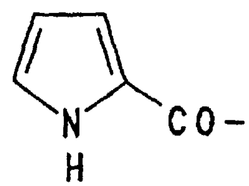 | 526 |
| 2.9 | 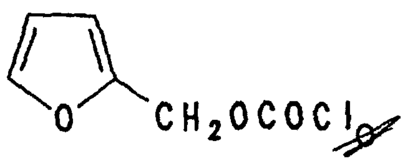 | 1b | -$C_2H_5$ | 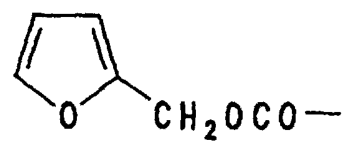 | 557 |
| 2.10 | 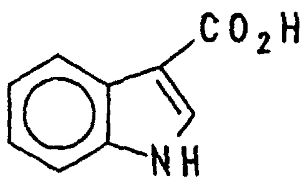 | 2b | $C_2H_5$ | 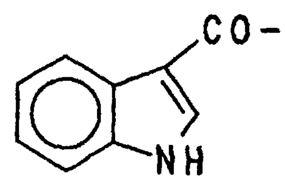 | 576 |

| | | | | | |
|---|---|---|---|---|---|
| 2.11 | 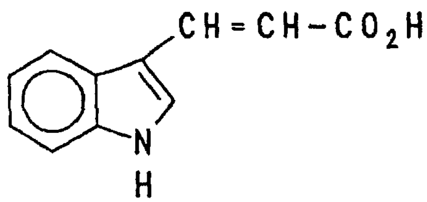 | 2b | $-C_2H_5$ | 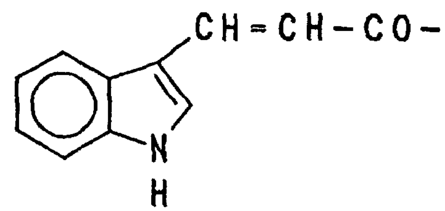 | 602 |

Liste 2

A) Folgende Carbonsäuren der Formel IV bzw. deren aktivierte Derivate, kommen als Ausgangssubstanzen beispielsweise in Frage:

1. Benzoesäuren und Derivate
Benzoesäure;

2. Heteroaromatische Carbonsäuren
2-, 3- oder 4-Pyridincarbonsäure; Thiophen-2- oder -3-carbonsäure; Pyrrol-2-carbonsäure;

3. Aryl- und Hetarylessigsäuren und Analoge bzw. Homologe

a.) nicht annelierte Säuren
Phenylessigsäure; 3-Phenyl-propionsäure; D,L-2-Phenyl-propionsäure; (S)-(+)-2-Phenylpropionsäure; (R)-(-)-2-Phenyfpropionsäure; 4-Phenyl-buttersäure; Phenylpropiolsäure; (4-Phenyl)buttersäure; 2-Thienylessigsäure; 3-Thienylessigsäure; Furylessigsäure; 2-, 3- oder 4-Pyridylessigsäure; 3-(2-Thienyl)propionsäuren; 3-(2-Furyl)propionsäure;

b.) Annelierte Säuren
Indol-2-carbonsäure; Indol-3-carbonsäure; Indol-4-carbonsäure;
3-Indolyl-essigsäure; 2-Indolyl-essigsäure;

B) Folgende Chlorameisensäureester (Halogenformate) der Formel III kommen als Ausgangssubstanzen beispielsweise in Frage:
Chlorameisensäure-phenylester
Chlorameisensäure-benzylester.

## Patentansprüche

**1.** Corticoid-17-alkylcarbonat-21-carbonsäure- und -kohlensäureester der Formel I

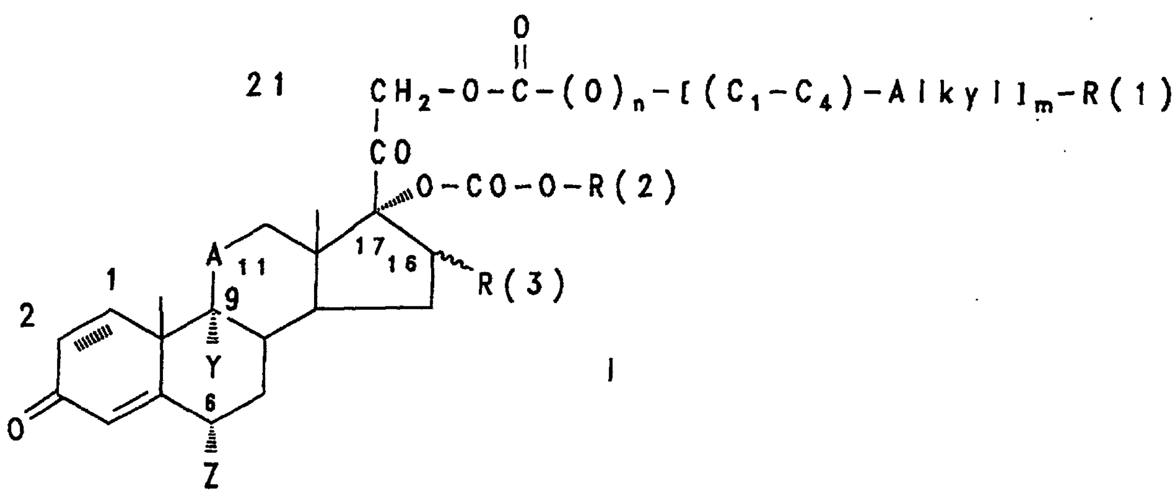

I

in welcher bedeuten:

A CHOH und CHCl in beliebiger sterischer Anordnung, $CH_2$, C=O, 9(11)-Doppelbindung;
Y Wasserstoff, Fluor oder Chlor;
Z Wasserstoff, Fluor oder Methyl;

R(1) Phenyl, Pyridyl, Thienyl, Pyrrolyl, Furyl, Indolyl oder Fluorenyl, ($C_1$-$C_4$)-Alkyl gesättigt, einfach oder mehrfach ungesättigt;
n Null oder 1,
m Null oder 1,
R(2) lineares oder verzweigtes ($C_1$-$C_8$)-Alkyl, $-(CH_2)_2-OCH_3$
R(3) Wasserstoff, α- oder β-Methyl.

**2.** Verbindung der Formel I nach Anspruch 1, **dadurch gekennzeichnet, daß** darin sind:

R(1), A, Y, Z und R(3)
wie in Anspruch 1 definiert; und
R(2) lineares oder verzweigtes ($C_1$-$C_5$)-Alkyl, $-(CH_2)_2-OCH_3$.

**3.** Verbindung der Formel I nach Anspruch 1 oder 2, **dadurch gekennzeichnet, dass** R(1) gleich Phenyl ist.

**4.** Verbindung der Formel I nach Anspruch 1, **dadurch gekennzeichnet, dass** es sich um

Prednisolon-17-ethylcarbonat-21-benzoat,
Prednisolon-17-ethylcarbonat-21-phenylacetat,
Prednisolon-17-ethylcarbonat-21-(3)-phenylpropionat,
Prednisolon-17-ethylcarbonat-21-(thienyl-2-essigsäure)ester,
Prednisolon-17-ethylcarbonat-21-(thiophen-2-carbonsäure)ester,
Prednisolon-17-ethylcarbonat-21-(furan-2-carbonsäureester),
Prednisolon-17-n-propylcarbonat-21-(3)-phenylpropionat,
Prednisolon-17-n-propylcarbonat-21-phenylacetat,
Prednisolon-17-iso-propylcarbonat-21-phenylacetat,
Prednisolon-17-n-butylcarbonat-21-phenylacetat,
Betamethason-17-ethylcarbonat-21-(3)-phenyl-propionat,
Betamethason-17-ethylcarbonat-21-phenylacetat,
Betamethason-17-ethylcarbonat-21-(thienyl-2-essigsäure)ester,
Betamethason-17-ethylcarbonat-21-(furan-2-carbonsäureester),
Betamethason-17-ethylcarbonat-21-(indol-3-essigsäure)ester,
Prednisolon-17-ethylcarbonat-21-(indol-3-essigsäure)ester,
Dexamethason-17-ethylcarbonat-21-phenylacetat,
Hydrocortison-17-ethylcarbonat-21-phenylacetat,
Cortison-17-ethylcarbonat-21-phenylacetat,
6α-Methylprednisolon-17-ethylcarbonat-21-phenylacetat,
Prednison-17-ethylcarbonat-21-phenylacetat,
6α-Fluor-prednisolon-17-ethylcarbonat-21-phenylacetat,
6α-Fluor-dexamethason-17-ethylcarbonat-21-phenylacetat,
6α-Fluor-betamethason-17-ethylcarbonat-21-phenylacetat,
6α,16α-Dimethyl-prednisolon-17-ethylcarbonat-21-phenylacetat,
17α-Ethylcarbonat, 21-phenylacetat von Reichsteins Substanz S,
Beclomethason-17α-ethylcarbonat-21-phenylacetat,
6α-Methyl-9α-fluor-prednisolon-17-ethylcarbonat-21-phenylacetat,
Betamethason-17-n-propylcarbonat-21-phenylacetat,
Dexamethason-17-iso-propylcarbonat-21-phenylacetat,
Prednisolon-17-n-propylcarbonat-21-phenylacetat,
Prednisolon-17-iso-propylcarbonat-21-phenylacetat,
Prednisolon-17-n-butylcarbonat-21-phenylacetat,
Prednisolon-17-iso-butylcarbonat-21-phenylacetat,
Prednisolon-17-methoxyethylcarbonat-21-phenylacetat oder
Prednisolon- 17-ethylcarbonat-21-phenylcarbonat

handelt.

**5.** Verbindung der Formel I nach Anspruch 1, **dadurch gekennzeichnet, dass** es sich um Prednisolon-17-ethylcarbonat-21-phenylacetat oder Betamethason-17-ethylcarbonat-21-phenylacetat handelt.

**6.** Verfahren zum Herstellen einer Verbindung I nach Anspruch 1, **dadurch gekennzeichnet, daß** man

a) eine Verbindung der Formel II,

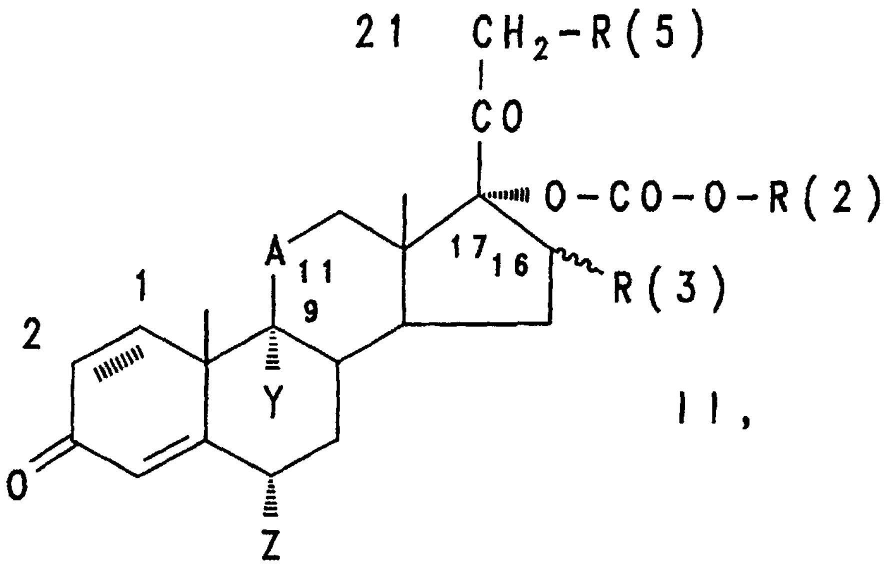

II,

in der R(5) gleich OH ist und die übrigen Substituenten die in Anspruch 1 angegebenen Bedeutungen haben,

a 1) mit einer aktivierten Carbonsäure der Formel III, vorzugsweise einem Halogenid oder Anhydrid oder Azolid,

$$R(6)\text{-}CO\text{-}(O)_n\text{-}[(C_1\text{-}C_4)\text{-Alkyl}]_m\text{-}R(1) \qquad III$$

umsetzt, wobei bedeuten:

n Null,
m Null oder 1, und

$[(C_1\text{-}C_4)\text{-Alkyl}]$ und R(1) die in Anspruch 1 angegebenen Bedeutungen haben und

R(6) Cl, Br, $O[\text{-}CO\text{-}(O)_n\text{-}[(C_1\text{-}C_4)\text{-Alkyl}]_m\text{-}R(1)]_1$-, $-OC(O)CF_3$ oder ein anderes aktiviertes Säureradikal, oder

a 2) mit einem Halogenformat der Formel III,
in der

n 1,
m Null oder 1,

$[(C_1\text{-}C_4)\text{-Alkyl}]$ und R(1) die oben angegebenen Bedeutungen haben und R(6) Cl, Br, J bedeuten, oder

a 3) mit einer Carbonsäure der Formel III selbst, in der

R(6) OH und
n Null sind,

und die weiteren Substituenten bei Formel III angegeben sind,

in Gegenwart Wasser abspaltender Reagentien (DCC etc.) umsetzt oder daß man

b) Verbindungen der Formel II,

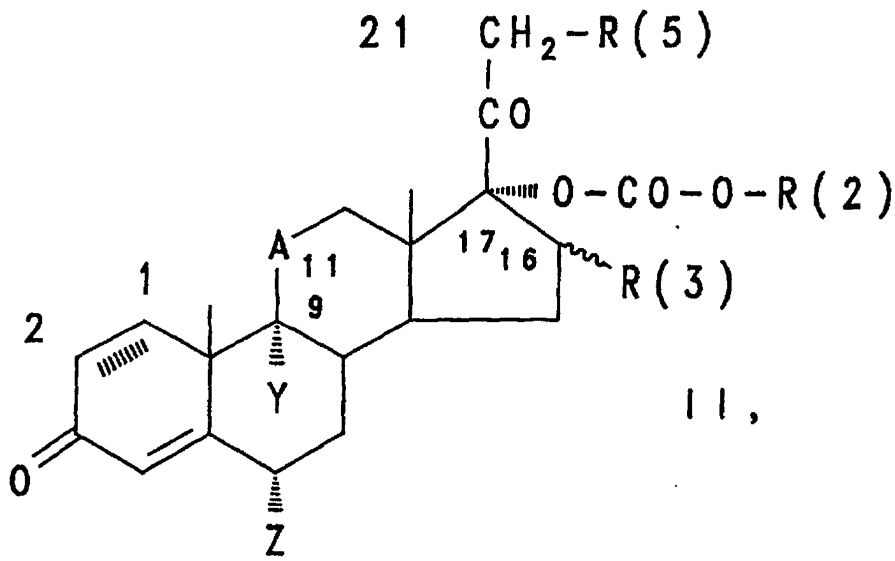

II,

in der R(5) = Br, J, eine Sulfonsäurearyl- oder -alkylestergruppierung ist und die weiteren Substituenten die bei Formel I angegebenen Bedeutung haben,
mit einem Salz, vorzugsweise K- oder Na-Salz oder einem Trialkylammoniumsalz, einer Carbonsäure der Formel III,

$$R(6)\text{-CO-}(O)_n\text{-}[(C_1\text{-}C_4)\text{-Alkyl}]_m\text{-}R(1) \qquad III$$

in der

R(6) -[$O^-Me^+$] und
n Null bedeuten,

und die weiteren Substituenten die bei Formel III angegebenen Bedeutungen haben,
umsetzt,

wobei Me vorzugsweise das Kation eines Alkalisalzes oder eines Trialkylammoniumsalzes ist.

7. Medikament zur Behandlung von Dermatosen insbesondere entzündlichen und allergischen, **gekennzeichnet durch** einen wirksamen Gehalt einer Verbindung I nach Anspruch 1.

8. Verwendung einer Verbindung I nach Anspruch 1 zum Herstellen eines Medikaments zur Behandlung von Dermatosen.

## Claims

1. A corticoid 17-alkyl carbonate 21-carboxylic or carbonic ester of the formula I

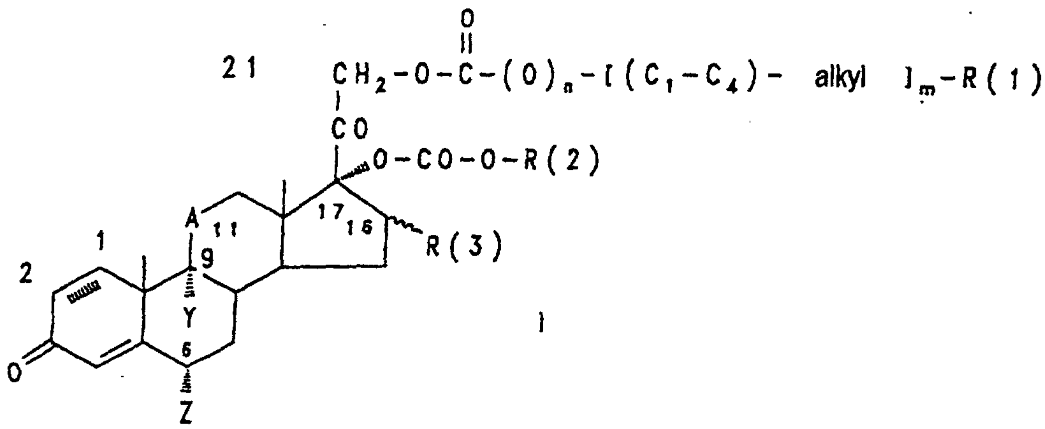

I

in which:

A is CHOH and CHCl in arbitrary stearic arrangement, $CH_2$, C = O or 9(11) double bond;
Y is hydrogen, fluorine or chlorine;
Z is hydrogen, fluorine or methyl;
R(1) is phenyl, pyridyl, thienyl, pyrrolyl, furyl, indolyl or fluorenyl, ($C_1$-$C_4$)-alkyl is saturated or unsaturated once or more than once;
n is zero or 1,
m is zero or 1,
R(2) is linear or branched ($C_1$-$C_8$)-alkyl or $-(CH_2)_2-OCH_3$,
R(3) is hydrogen or α- or β-methyl.

**2.** A compound of the formula I as claimed in claim 1, wherein R(1), A, Y, Z and R(3) are defined as in claim 1, and R(2) is linear or branched ($C_1$-$C_5$)-alkyl or $-(CH_2)_2-OCH_3$.

**3.** A compound of the formula I as claimed in claim 1 or 2, wherein R(1) is phenyl.

**4.** A compound of the formula I as claimed in claim 1, wherein the compound is

prednisolone 17-ethyl carbonate 21-benzoate,
prednisolone 17-ethyl carbonate 21-phenylacetate,
prednisolone 17-ethyl carbonate 21-(3)-phenylpropionate,
prednisolone 17-ethyl carbonate 21-(thienyl-2-acetic) ester,
prednisolone 17-ethyl carbonate 21-(thiophene-2-carboxylic) ester,
prednisolone 17-ethyl carbonate 21-(furan-2-carboxylic ester),
prednisolone 17-n-propyl carbonate 21-(3)-phenylpropionate,
prednisolone 17-n-propyl carbonate 21-phenylacetate,
prednisolone 17-isopropyl carbonate 21-phenylacetate,
prednisolone 17-n-butyl carbonate 21-phenylacetate,
betamethasone 17-ethyl carbonate 21-(3)-phenylpropionate,
betamethasone 17-ethyl carbonate 21-phenylacetate,
betamethasone 17-ethyl carbonate 21-(thienyl-2-acetic) ester,
betamethasone 17-ethyl carbonate 21-(furan-2-carboxylic ester),
betamethasone 17-ethyl carbonate 21-(indole-3-acetic) ester,
prednisolone 17-ethyl carbonate 21-(indole-3-acetic) ester,
dexamethasone 17-ethyl carbonate 21-phenylacetate,
hydrocortisone 17-ethyl carbonate 21-phenylacetate,
cortisone 17-ethyl carbonate 21-phenylacetate,
6α-methylprednisolone 17-ethyl carbonate 21-phenylacetate,
prednisone 17-ethyl carbonate 21-phenylacetate,
6α-fluoroprednisolone 17-ethyl carbonate 21-phenylacetate,
6α-fluorodexamethasone 17-ethyl carbonate 21-phenylacetate,

6α-fluorobetamethasone 17-ethyl carbonate 21-phenyl acetate,
6α,16α-dimethylprednisolone 17-ethyl carbonate 21-phenylacetate,
17α-ethyl carbonate, 21-phenylacetate of Reichstein's substance S,
beclomethasone 17α-ethyl carbonate 21-phenylacetate,
6α-methyl-9α-fluoroprednisolone 17-ethyl carbonate 21-phenylacetate,
betamethasone 17-n-propyl carbonate 21-phenylacetate,
dexamethasone 17-isopropyl carbonate 21-phenylacetate,
prednisolone 17-n-propyl carbonate 21-phenylacetate,
prednisolone 17-isopropyl carbonate 21-phenylacetate,
prednisolone 17-n-butyl carbonate 21-phenylacetate,
prednisolone 17-isobutyl carbonate 21-phenylacetate,
prednisolone 17-methoxyethyl carbonate 21-phenylacetate, or
prednisolone 17-ethyl carbonate 21-phenylcarbonate.

**5.** A compound of the formula I as claimed in claim 1, which is prednisolone 17-ethyl carbonate 21-phenylacetate or betamethasone 17-ethyl carbonate 21-phenylacetate.

**6.** A process for preparing a compound I as claimed in claim 1, wherein

a) a compound of the formula II,

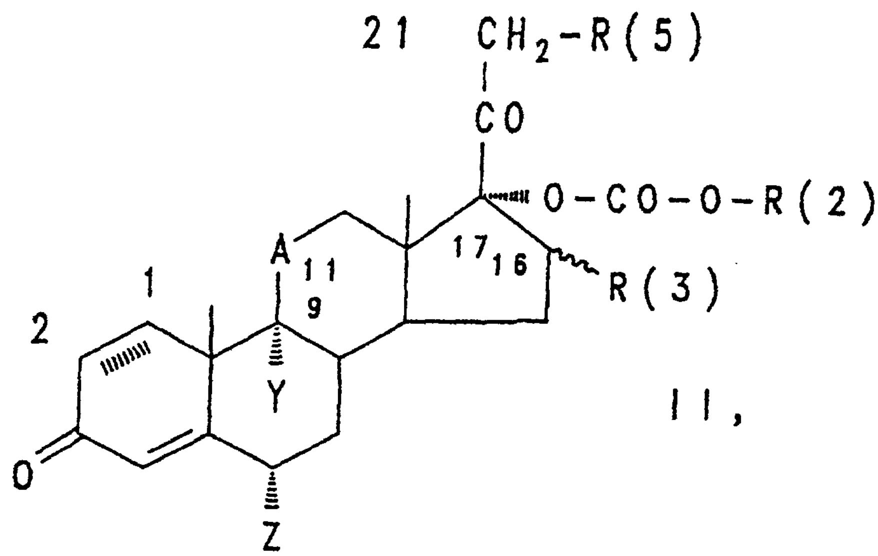

II,

in which R(5) is OH and the remaining substituents have the meanings given in claim 1,

a 1) is reacted with an activated carboxylic acid of formula III, preferably a halide or anhydride or azolide,

$$R(6)\text{-CO-}(O)_n\text{-}[(C_1\text{-}C_4)\text{-alkyl}]_m\text{-}R(1) \qquad III$$

where:

n is zero,
m is zero or 1, and

$[(C_1-C_4)$-alkyl] and R(1) have the meanings given in claim 1, and

R(6) is Cl, Br, $O[\text{-CO-}(O)_n\text{-}[(C_1\text{-}C_4)\text{-alkyl}]_m\text{- } R(1)]_1$, $-OC(O)CF_3$, or another activated acid radical, or

a 2) is reacted with a haloformate of formula III, in which

n is 1,
m is zero or 1,

[($C_1$-$C_4$)-alkyl] and R(1) have the abovementioned meanings, and R(6) is Cl, Br or I, or

a 3) is reacted with a carboxylic acid of the formula III itself, in which R(6) is OH, and

n is zero,

and the other substituents are given in formula III, in the presence of water-eliminating reagents (DCC, etc.), or in which

b) compounds of the formula II,

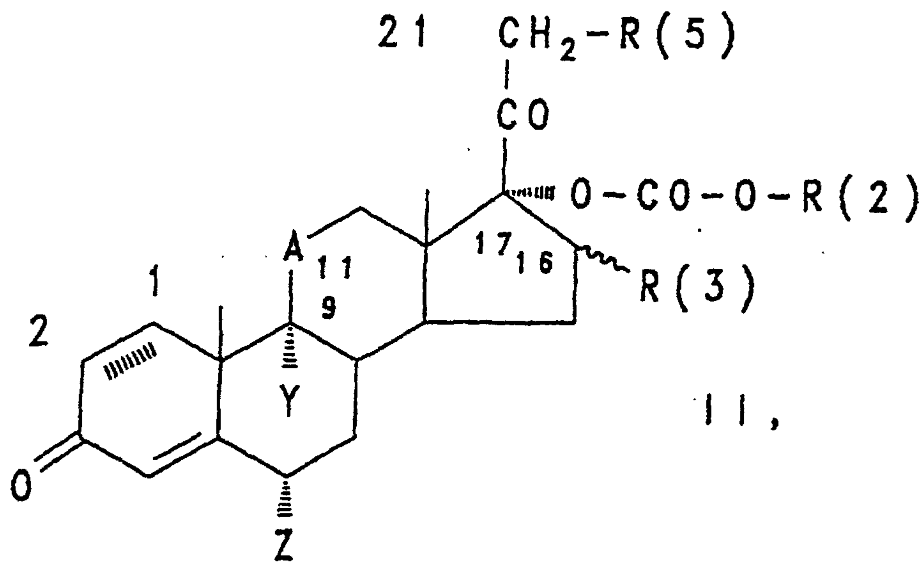

II,

in which R(5) = Br, I or a sulfonic aryl or alkyl ester group, and the remaining substituents have the meanings given in formula I, is reacted with a salt, preferably K or Na salt, or a trialkylammonium salt, of a carboxylic acid of the formula III,

$$R(6)\text{-CO-(O)}_n\text{-[(C}_1\text{-C}_4\text{)-alkyl]}_m\text{-R(1)} \qquad \text{III}$$

in which

R(6) is -($O^-Me^+$), and
n is zero,

and the remaining substituents have the meanings given in formula III,

where Me is preferably the cation of an alkali metal salt or of a trialkylammonium salt.

**7.** A pharmaceutical for treating dermatoses, in particular those which are inflammatory and allergic, which has an effective content of a compound I as claimed in claim 1.

**8.** Use of a compound I as claimed in claim 1 for preparing a pharmaceutical for treating dermatoses.

## Revendications

**1.** 17-alkylcarbonate-21-carboxylate et 21-carbonate de corticoïde de formule I

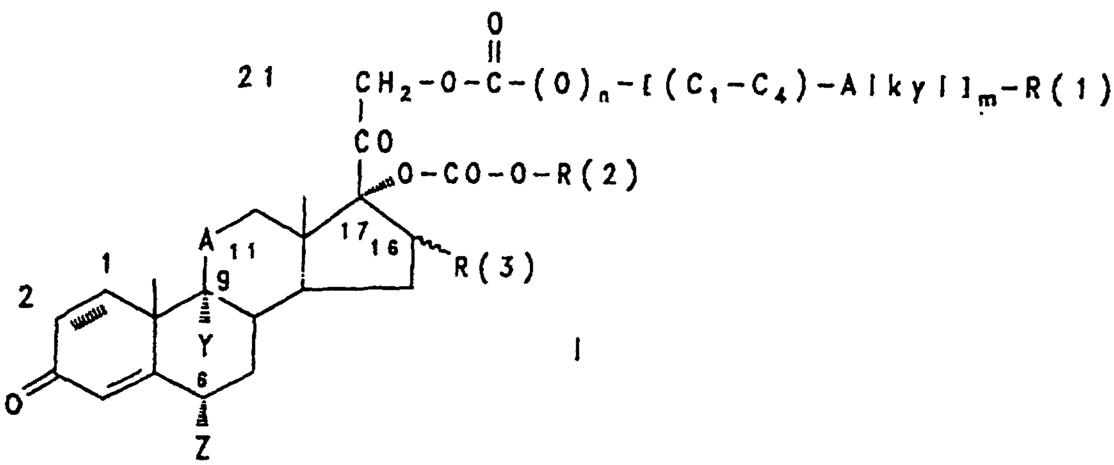

I

dans laquelle

| | |
|---|---|
| A | représente un groupe CHOH ou CHCl en une configuration stérique quelconque, $CH_2$ ou C=O, ou une double liaison en 9(11); |
| Y | représente un atome d'hydrogène, de fluor ou de chlore; |
| Z | représente un atome d'hydrogène ou de fluor ou un groupe méthyle; |
| R(1) | représente un groupe phényle, pyridyle, thiényle, pyrrolyle, indolyle ou fluorényle; |
| $(C_1$-$C_4)$-Alkyl | est un groupe alkyle en $C_1$-$C_4$, saturé ou insaturé une ou plusieurs fois; |
| n | est 0 ou 1 ; |
| m | est 0 ou 1; |
| R(2) | représente un groupe alkyle en $C_1$-$C_8$ linéaire ou ramifié ou $-(CH_2)_2-OCH_3$; |
| R(3) | représente un atome d'hydrogène ou un groupe α- ou β-méthyle. |

**2.** Composé de formule I selon la revendication 1, **caractérisé en ce que** R(1), A, Y, Z et R(3) ont la définition donnée dans la revendication 1; et R(2) représente un groupe alkyle en $C_1$-$C_5$ linéaire ou ramifié ou $-(CH_2)_2-OCH_3$.

**3.** Composé de formule I selon la revendication 1 ou 2, **caractérisé en ce que** R(1) représente un groupe phényle.

**4.** Composé de formule I selon la revendication 1, **caractérisé en ce qu'**il s'agit

du 17-éthylcarbonate-21-benzoate de prednisolone,
du 17-éthylcarbonate-21-phénylacétate de prednisolone,
du 17-éthylcarbonate-21-(3)-phénylpropionate de prednisolone,
du 17-éthylcarbonate-21-(thiényl-2-acétate) de prednisolone,
du 17-éthylcarbonate-21-(thiophène-2-carboxylate) de prednisolone,
du 17-éthylcarbonate-21-(furane-2-carboxylate) de prednisolone,
du 17-n-propylcarbonate-21-(3)-phénylpropionate de prednisolone,
du 17-n-propylcarbonate-21-phénylacétate de prednisolone,
du 17-isopropylcarbonate-21-phénylacétate de prednisolone,
du 17-n-butylcarbonate-21-phénylacétate de prednisolone,
du 17-éthylcarbonate-21-(3)-phénylpropionate de bétaméthasone,
du 17-éthylcarbonate-21-phénylacétate de bétaméthasone,
du 17-éthylcarbonate-21-(thiényl-2-acétate) de bétaméthasone,

du 17-éthylcarbonate-21-(furane-2-carboxylate) de bétaméthasone,
du 17-éthylcarbonate-21-(indole-3-acétate) de bétaméthasone,
du 17-éthylcarbonate-21-(indole-3-acétate) de prednisolone,
du 17-éthylcarbonate-21-phénylacétate de dexaméthasone,
du 17-éthylcarbonate-21-phénylacétate d'hydrocortisone,
du 17-éthylcarbonate-21-phénylacétate de cortisone,
du 17-éthylcarbonate-21-phénylacétate de 6α-méthylprednisolone,
du 17-éthylcarbonate-21-phénylacétate de prednisone,
du 17-éthylcarbonate-21-phénylacétate de 6α-fluoroprednisolone,
du 17-éthylcarbonate-21-phénylacétate de 6α-fluorodexaméthasone,
du 17-éthylcarbonate-21-phénylacétate de 6α-fluorobétaméthasone,
du 17-éthylcarbonate-21-phénylacétate de 6α,16α-diméthylprednisolone,
du 17α-éthylcarbonate, 21-phénylacétate de la substance S de Reichstein,
du 17α-éthylcarbonate-21-phénylacétate de béclométhasone,
du 17-éthylcarbonate-21-phénylacétate de 6α-méthyl-9α-fluoroprednisolone,
du 17-n-propylcarbonate-21-phénylacétate de bétaméthasone,
du 17-isopropylcarbonate-21-phénylacétate de dexaméthasone,
du 17-n-propylcarbonate-21-phénylacétate de prednisolone,
du 17-isopropylcarbonate-21-phénylacétate de prednisolone,
du 17-n-butylcarbonate-21-phénylacétate de prednisolone,
du 17-isobutylcarbonate-21-phénylacétate de prednisolone,
du 17-méthoxyéthylcarbonate-21-phénylacétate de prednisolone ou
du 17-éthylcarbonate-21-phénylcarbonate de prednisolone.

**5.** Composé de formule I selon la revendication 1, **caractérisé en ce qu'**il s'agit du 17-éthylcarbonate-21-phénylacétate de prednisolone ou du 17-éthylcarbonate-21-phénylacétate de bétaméthasone.

**6.** Procédé de préparation d'un composé I selon la revendication 1, **caractérisé en ce que**

a) on fait réagir un composé de formule II

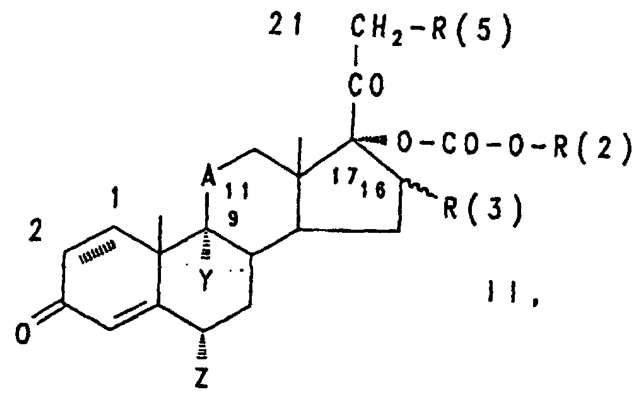

II,

dans laquelle R(5) représente un groupe OH et les autres substituants ont la signification indiquée dans la revendication 1,

(a1) avec un acide carboxylique activé de formule III, de préférence un halogénure, un anhydride ou un azolide,

$$R(6)\text{-}CO\text{-}(O)_n\text{-}[(C_1\text{-}C_4)\text{-}Alkyl]_m\text{-}R(1) \qquad III$$

dans laquelle:
n est 0,

m est 0 ou 1, et
$[(C_1-C_4)$-Alkyl] et R(1) ont la signification indiquée dans la revendication 1, et
R(6) représente Cl, Br, $O[-CO-(O)_n-[(C_1-C_4)$-Alkyl$]_m$-R(1)$]_1$-, $-OC(O)CF_3$ ou un autre radical d'acide activé, ou
a2) avec un halogénoformate de formule III dans laquelle
n est 1,
m est 0 ou 1, et
$[(C_1-C_4)$-Alkyl] et R(1) ont la signification indiquée dans la revendication 1, et
R(6) représente Cl, Br ou I, ou
a3) avec un acide carboxylique de formule III dans laquelle
R(6) est OH et n est 0, et les autres substituants sont définis pour la formule III,

en présence de réactifs qui éliminent de l'eau (DCC, etc.),
ou **en ce que**
b) on fait réagir des composés de formule II

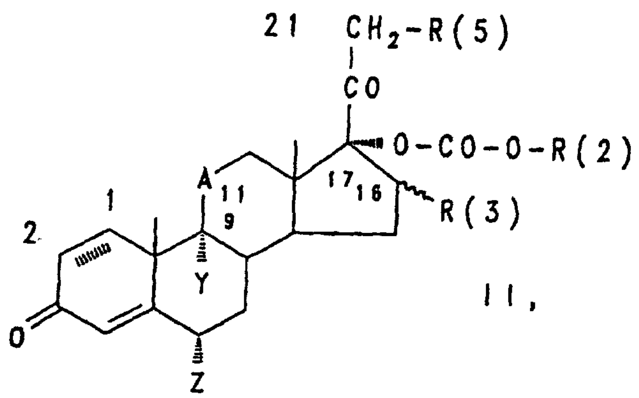

II,

dans laquelle R(5) = Br, I ou un groupe sulfonate d'aryle ou d'alkyle et les autres substituants ont la signification indiquée pour la formule I,
avec un sel, de préférence un sel de K ou Na ou un sel de trialkylammonium, d'un acide carboxylique de formule III

$$R(6)\text{-}CO\text{-}(O)_n\text{-}[(C_1\text{-}C_4)\text{-Alkyl}]_m\text{-}R(1) \qquad III$$

dans laquelle:
R(6) représente $-[O^-Me^+]$ et
n est 0,
et les autres substituants ont la signification indiquée pour la formule III, Me étant de préférence le cation d'un sel de métal alcalin ou d'un sel de trialkylammonium.

7. Médicament pour le traitement de dermatoses, en particulier inflammatoires et allergiques, **caractérisé en ce qu'**il contient une quantité efficace d'un composé I selon la revendication 1.

8. Utilisation d'un composé I selon la revendication 1 pour la préparation d'un médicament destiné au traitement de dermatoses.